# EUROPEAN PATENT APPLICATION

(11) **EP 4 226 933 A1**
(43) Date of publication of application: **16.08.2023**
(21) Application number: 21872927.5
(22) Date of filing: 24.09.2021
(51) Int. Cl.: A61K 38/16, A61K 47/68, A61K 38/17, A61K 38/26, A61P 25/00, A61P 37/00

(54) **THERAPEUTIC USE OF LONG-ACTING CONJUGATE OF TRIPLE AGONIST ACTING ON ALL OF GLUCAGON, GLP-1 AND GIP RECEPTORS AGAINST MULTIPLE SCLEROSIS**

(30) Priority: 25.09.2020 KR 20200125197
(71) Applicant: Hanmi Pharm. Co., Ltd., Hwaseong-si, Gyeonggi-do 18536 (KR)
(72) Inventor: LEE, Sang Don, Hwaseong-si, Gyeonggi-do 18469 (KR); KIM, Jeong A, Hwaseong-si, Gyeonggi-do 18469 (KR); LEE, Sang Hyun, Hwaseong-si, Gyeonggi-do 18469 (KR); LEE, Jong-Soo, Hwaseong-si, Gyeonggi-do 18469 (KR)
(74) Representative: Mewburn Ellis LLP
(86) International application number: PCT/KR2021/013008
(87) International publication number: WO 2022/065899

(57) **Abstract**

The present invention relates to therapeutic uses against multiple sclerosis, of a triple agonist and/or a long-acting conjugate thereof, acting on all of glucagon, GLP-1 and GIP receptors. Patients' options can be broadened by expanding the category of drugs applicable to multiple sclerosis and patients' convenience can be increased by significantly increasing blood half-life.

## Description

### [Technical Field]

The present invention relates to preventive or therapeutic uses, for multiple sclerosis, of a triple agonist having activities for all of glucagon, GLP-1, and GIP receptors and/or a conjugate thereof.

### [Background Art]

Multiple sclerosis is a chronic neuro-immunological disease that occurs in the central nervous system, including the brain and spinal cord, and is a representative example of demyelinating diseases. Under normal circumstances, myelin sheaths surrounding neuronal axons act as an electric insulator for neuronal signals, enabling rapid neuronal signal conduction. The exposure of axons due to the demyelination causes abnormalities in the conduction of neuronal signals, resulting in the death of neurons. Multiple sclerosis is classified into four types according to the progression of the disease: relapsing/remitting multiple sclerosis (RRMS), secondary progressive multiple sclerosis (SPMS), primary progressive multiple sclerosis (PPMS), and progressive relapsing multiple sclerosis (PRMS). In addition, 85% of early-stage multiple sclerosis patients are diagnosed with relapsing/remitting multiple sclerosis. Multiple sclerosis is developed by the infiltration of inflammatory cells to the central nervous system, which is known to be caused by ab autoimmune response, and it is known that genetic and environmental factors affect the susceptibility to the disease. In particular, there are many reports that the body mass index affects the incidence of multiple sclerosis, which is approximately doubled for obesity (Neurology. 2009; 73(19):1543-50, The EnvIMS study. Mult Scler. 2014, Obes Res Clin Pract. 2014; 8(5): e435-47).

The US Food and Drug Administration (FDA) has approved several treatments for multiple sclerosis, including interferon beta, glartiramer acetate, dimethyl fumarate, fingolimod, sphingosine 1 phosphate receptor modulator, natalizumab (alpha-4 integrin monoclonal antibody), ocrelizumab (CD-20 nomoclonal antibody), and the like. These medicines, except for ocrelizumab, are used only to treat relapsing/remitting multiple sclerosis or to delay the diseases or alleviate symptoms. Ocrelizumab is the first medicine approved for primary progressive multiple sclerosis, but further clinical trials are needed to address its side effects on infection, safety during long-term administration, and cancer incidence. Medicines that are developed in the future need to address both the relief of side effects of currently used medicines and the stop of disease progression in progressive multiple sclerosis.

Meanwhile, recent studies have demonstrated the efficacy of substances in multiple sclerosis, which have mechanisms different from existing medicines. Out of these, glucagon-like peptide-1 (GLP-1), a typical incretin hormone, showed good effect in pre-clinical studies. It was confirmed that liraglutide, a GLP-1 analog, delayed the onset of the disease in an experimentally induced multiple sclerosis rat model, and had a symptom relieving effect by lowering the score of clinical symptoms occurring at the time of onset (Front Pharmacol. 2016 Nov 18; 7:433). It was also confirmed that exendin-4, a GLP-1 receptor agonist, had a symptom alleviating effect in a relapsing/remitting multiple sclerosis mouse model that was experimentally induced, and the effect was similar to that of fingolimod. In particular, administration of exendin-4 reduced the levels of inflammatory cytokines that increase during demyelination in this model (Mol Neurobiol. 2018 Apr; 55(4):3007-3020).

Approximately 60-70% of diabetic patients have some degree of neurological damage, specifically neuropathies that result in impaired sensation in the hands and/or feet, slowed gastric motility, or carpal tunnel syndrome. There is currently no therapy that has been proven to reverse the neurological damage caused by prolonged hyperglycemia and the associated metabolic disturbances. GLP-1 expression has been identified in neurons in the nodose ganglion, suggesting a role for GLP-1 in peripheral neurotransmission. It was revealed that in a rodent model of pyridoxine-induced peripheral neuropathy in non-diabetic rodents, GLP-1 and s.c. exendin-4 partially protected against several pyridoxine-induced functional and morphological defects and to facilitate normalization of axonal size (Exp Neurol. 2007 Feb; 203(2):293-301). Nevertheless, there is still a great need for practical medicines capable of treating the neuropathy.

### [Disclosure]

### [Technical Problem]

Practical and effective medicines for multiple sclerosis as neuropathy, a type of various neurodegenerative diseases, and multiple sclerosis caused by complications of diabetes in a diabetic patient are not sufficient so far, and thus there is a need for continuous development of medicines.

### [Technical Solution]

An aspect of the present invention is to provide a pharmaceutical composition for prevention or treatment of multiple sclerosis, the pharmaceutical composition including a peptide having activities for glucagon, glucagon-like peptide-1 (GLP-1), and Glucose-dependent insulinotropic polypeptide (GIP) receptors or a long-acting conjugate of the peptide.

Another aspect of the present invention is to provide a method for prevention or treatment of multiple sclerosis, the method including administering to a subject in need thereof a composition containing the peptide or a long-acting conjugate of the peptide.

Still another aspect of the present invention is to provide use of a composition containing the peptide or a long-acting conjugate of the peptide in the preparation of a medicine for prevention or treatment of multiple sclerosis.

### [Advantageous Effects]

The triple agonists or long-acting conjugates thereof according to the present invention have activities for glucagon, glucagon-like peptide-1 (GLP-1), and glucose-dependent insulinotropic polypeptide (GIP) receptors and thus can expand the category of drugs, which have been applicable to multiple sclerosis until now, to thereby widen the option of patients and significantly increase the blood half-life, leading to an improvement in the convenience of patients.

### [Brief Description of Drawings]

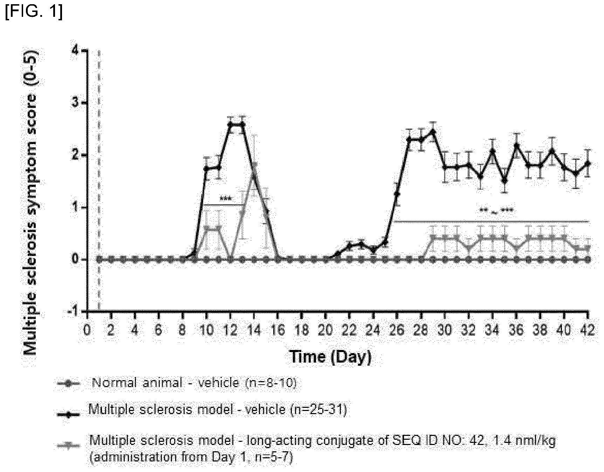

FIG. 1 shows multiple sclerosis symptom scores, in a multiple sclerosis mouse model induced by PLP/PTX, after a long-acting conjugate of a triple agonist was administered from one day after the introduction of the model, for the purpose of prevention (*p<0.05, **p<0.01, ***p<0.001, vs. multiple sclerosis model vehicle control by two-way ANOVA).

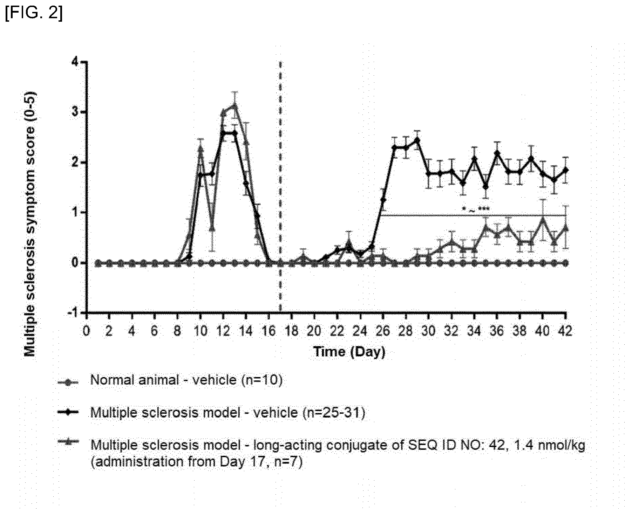

FIG. 2 shows multiple sclerosis symptom scores, in a multiple sclerosis mouse model induced by PLP/PTX, after a long-acting conjugate of a triple agonist was administered from one day after the introduction of the model, for the purpose of treatment (*p<0.05, **p<0.01, ***p<0.001, vs. multiple sclerosis model vehicle control by two-way ANOVA).

### [Best Mode for Carrying Out the Invention]

In accordance with an aspect of the present invention, there is provided a pharmaceutical composition for prevention or treatment of multiple sclerosis, the pharmaceutical composition containing a peptide having activities for glucagon, glucagon-like peptide-1 (GLP-1), and glucose-dependent insulinotropic polypeptide (GIP) receptors.

In an embodiment, the pharmaceutical composition for prevention or treatment of multiple sclerosis contains: a pharmaceutically acceptable vehicle; and a pharmaceutically effective amount of a peptide containing an amino acid sequence of any one of SEQ ID NOS: 1 to 102.

In the pharmaceutical composition according to any one of the previous embodiments, the peptide is in the form of a long-acting conjugate, wherein the long-acting conjugate is represented by Formula 1 below:

[Formula 1] X-L-F

wherein X represents a peptide containing an amino sequence of any one of SEQ ID NOS: 1 to 102;
L represents a linker containing ethylene glycol repeat units;
F represents an immunoglobulin Fc region; and
"-" symbols represent covalent linkages between X and L and between L and F, respectively.

In the composition according to any one of the previous embodiments, the C-terminus of the peptide is amidated.

In the composition according to any one of the previous embodiments, the C-terminus of the peptide is amidated or has a free carboxyl group (-COOH).

In the composition according to any one of the previous embodiments, the peptide contains an amino acid sequence selected from the group consisting of SEQ ID NOS: 21, 22, 42, 43, 50, 64, 66, 67, 70, 71, 76, 77, 96, 97, and 100.

In the composition according to any one of the previous embodiments, the peptide contains an amino acid sequence selected from the group consisting of SEQ ID NOS: 21, 22, 42, 43, 50, 66, 67, 77, 96, 97, and 100.

In the composition according to any one of the previous embodiments, the peptide contains an amino acid sequence selected from the group consisting of SEQ ID NOS: 21, 22, 42, 43, 50, 77, and 96.

In the composition according to any one of the previous embodiments, the amino acids at positions 16 and 20 form the N-terminus in the peptide sequence form a ring.

In the composition according to any one of the previous embodiments, L is polyethylene glycol.

In the composition according to any one of the previous embodiments, the formula weight of an ethylene glycol repeat unit moiety is in a range of 1 to 100 kDa.

In the composition according to any one of the previous embodiments, F is an IgG Fc region.

In the composition according to any one of the previous embodiments, the immunoglobulin Fc region is aglycosylated.

In the composition according to any one of the previous embodiments, the immunoglobulin Fc region is selected from the group consisting of: (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination between one or at least two domains among a CH1 domain, a CH2 domain, a CH3 domain, and a CH4 domain and an immunoglobulin hinge region or a part of the hinge region; and (f) a dimer between each domain of a heavy chain constant region and a light chain constant region.

In the composition according to any one of the previous embodiments, the immunoglobulin Fc region is a native Fc derivative in which a region capable of forming a disulfide bond is deleted, some amino acids in the N-terminus is deleted, a methionine residue is added to the N-terminus, a complement-binding site is deleted, or an antibody dependent cell mediated cytotoxicity (ADCC) site is deleted.

In the composition according to any one of the previous embodiments, the immunoglobulin Fc region is derived from IgG, IgA, IgD, IgE, or IgM.

In the composition according to any one of the previous embodiments, the immunoglobulin Fc region is a hybrid of domains with different origins derived from an immunoglobulin selected from the group consisting of IgG, IgA, IgD, IgE, and IgM.

In the composition according to any one of the previous embodiments, the immunoglobulin Fc region has a dimeric form.

In the composition according to any one of the previous embodiments, the immunoglobulin Fc region is a dimer consisting of two polypeptide chains, and one end of L is linked only to one of the two polypeptide chains.

In the composition according to any one of the previous embodiments, the immunoglobulin Fc region is an IgG4 Fc region.

In the composition according to any one of the previous embodiments, the immunoglobulin Fc region is an aglycosylated Fc region derived from human IgG4.

In the composition according to any one of the previous embodiments, the immunoglobulin Fc region has a structure in which two polypeptide chains are linked through a disulfide bond, wherein two polypeptide chains are linked through only a nitrogen atom of one of the two chains.

In the composition according to any one of the previous embodiments, the immunoglobulin Fc region includes a monomer of the amino acid sequence of SEQ ID NO: 123.

In the composition according to any one of the previous embodiments, the immunoglobulin Fc region is a homodimer of monomers of the amino acid sequence of SEQ ID NO: 123.

In the composition according to any one of the previous embodiments, the immunoglobulin Fc region is linked through a nitrogen atom of proline at the N-terminus.

In the composition according to any one of the previous embodiments, F, which is an immunoglobulin Fc region, and X are aglycosylated.

In the composition according to any one of the previous embodiments, the ethylene glycol repeat units correspond to [OCH₂CH₂]ₙ where n is a natural number, wherein n is determined such that the average molecular weight, for example, the number average molecular weight, of the [OCH₂CH₂]ₙ moiety in the peptide conjugate is 1 to 100 kDa.

In the composition according to any one of the previous embodiments, the value of n is determined such that the average molecular weight, for example, the number average molecular weight, of the [OCH₂CH₂]ₙ moiety in the peptide conjugate is 10 kDa.

In the composition according to any one of the previous embodiments, in the conjugate, one end of L is linked to F via a covalent linkage formed by reaction with an amine or thiol group of F, and the other end of L is linked to X via a covalent linkage formed by reaction with an amine or thiol group of X.

In the composition according to any one of the previous embodiments, L is polyethylene glycol.

In the composition according to any one of the previous embodiments, the multiple sclerosis is relapsing/remitting multiple sclerosis (RRMS), secondary progressive multiple sclerosis (SPMS), primary progressive multiple sclerosis (PPMS), or progressive relapsing multiple sclerosis (PRMS).

In accordance with another aspect of the present invention, there is provided a method for preventing or treating multiple sclerosis, the method including administering to a subject in need thereof the peptide or a composition containing the peptide.

In accordance with another aspect of the present invention, there is provided use of the peptide or a composition containing the peptide in the preparation of a medicine for prevention or treatment of multiple sclerosis.

In accordance with another aspect of the present invention, there is provided use of the peptide or a composition containing the same for the prevention or treatment of multiple sclerosis.

### [Mode for Carrying Out the Invention]

Hereinafter, the present invention will be described in detail.

Each description and embodiment disclosed in this disclosure may also be applied to other descriptions and embodiments. That is, all combinations of various elements disclosed in this disclosure fall within the scope of the present disclosure. Furthermore, the scope of the present invention is not limited by the specific description below.

Throughout the description herein, not only the typical one-letter and three-letter codes for naturally occurring amino acids, but also three-letter codes generally allowed for other amino acids, such as 2-aminoisobutyric acid (Aib), N-methylglycine (Sar), and α-methyl-glutamic acid, are used. The amino acids mentioned in abbreviations herein are described according to the IUPAC-IUB rules:
Herein, "Aib" may be used interchangeably with "2-aminoisobutyric acid" or "aminoisobutyric acid", and 2-aminoisobutyric acid and aminoisobutyric acid may be used interchangeably with each other.
alanine (Ala, A), arginine (Arg, R)
asparagine (Asn, N), aspartic acid (Asp, D)
cysteine (Cys, C), glutamic acid (Glu, E)
glutamine (Gln, Q), glycine (Gly, G)
histidine (His, H), isoleucine (Ile, I)
leucine (Leu, L), lysine (Lys, K)
methionine (Met, M), phenylalanine (Phe, F)
proline (Pro, P), serine (Ser, S)
threonine (Thr, T), tryptophan (Trp, W)
tyrosine (Tyr, Y), valine (Val, V)

In accordance with an aspect of the present invention, there is provided a pharmaceutical composition for prevention or treatment of multiple sclerosis, the pharmaceutical composition containing a peptide having activities for glucagon, glucagon-like peptide-1 (GLP-1), and glucose-dependent insulinotropic polypeptide (GIP) receptors.

In an embodiment, the peptide may contain an amino acid sequence of any one of SEQ ID NOS: 1 to 102.

In an embodiment, the pharmaceutical composition for prevention or treatment of multiple sclerosis contains: a pharmaceutically acceptable vehicle; and a pharmaceutically effective amount of a peptide containing an amino acid sequence of any one of SEQ ID NOS: 1 to 102.

The composition of the present invention may contain a pharmaceutically effective amount of a peptide having activities for glucagon, GLP-1, and GIP receptors, and specifically may contain a pharmaceutically effective amount of a peptide (a triple agonist) containing, consisting essentially of, or consisting of at least one sequence of the amino acid sequences of SEQ ID NOS: 1 to 102, but is not limited thereto.

The "peptide having activities for glucagon, GLP-1, and GIP receptors" may be used interchangeably with the term "triple agonist" or "triple agonist peptide".

The triple agonist of the present invention may include a peptide containing at least one of the amino acid sequences of SEQ ID NOS: 1 to 102. Alternatively, a peptide consisting essentially of or consisting of any one of the amino acid sequences of SEQ ID NOs: 1 to 102 may also be included in the triple agonist of the present invention, but is not limited thereto. The peptide containing an amino acid sequence of any one of SEQ ID NOS: 1 to 102 is an example of the peptide having activities for glucagon, GLP-1, and GIP receptors, but is not limited thereto.

This peptide encompasses various substances, such as various peptides, with significant levels of activities for glucagon, GLP-1, and GIP receptors.

The triple agonist having significant levels of activities for glucagon, GLP-1, and GIP receptors may exhibit *in vitro* activities for one or more receptors, specifically two or more receptors, and more specifically all three of the receptors among glucagon, GLP-1, and GIP receptors, of about 0.001% or more, about 0.01% or more, about 0.1% or more, about 1% or more, about 2% or more, about 3% or more, about 4% or more, about 5% or more, about 6% or more, about 7% or more, about 8% or more, about 9% or more, about 10% or more, about 20% or more, about 30% or more, about 40% or more, about 50% or more, about 60% or more, about 70% or more, about 80% or more, about 90% or more, about 100% or more, about 150% or more, or about 200% or more, compared with native ligands (native glucagon, native GLP-1, and native GIP) for the corresponding receptors, but significantly increasing ranges are included without limitation.

The activities for the receptors may include, for example, those cases where the *in vitro* activities for the receptor are about 0.001 % or more, about 0.01% or more, about 0.1% or more, about 1% or more, about 2% or more, about 3% or more, about 4% or more, about 5% or more, about 6% or more, about 7% or more, about 8% or more, about 9% or more, about 10% or more, about 20% or more, about 30% or more, about 40% or more, about 50 % or more, about 60% or more, about 70% or more, about 80% or more, about 90% or more, about 100% or more, or about 200% or more, compared with the native ligands. However, the activities for the receptors are not limited thereto.

As used herein, the term "about" refers to a range including ±0.5, ±0.4, ±0.3, ±0.2, ±0.1, and the like, and thus includes all of the values in the range equivalent or similar to those stated after this term, but is not limited thereto.

Methods for measuring the *in vitro* activities of triple agonists may refer to Test Example 1 herein, but are not particularly limited thereto.

Meanwhile, the peptide retains one or more, two or more, and specifically three of the following activities of i) to iii) below, specifically significant activities thereof:
i) activating the GLP-1 receptor; ii) activating the glucagon receptor; and iii) activating the GIP receptor.

In particular, the activation of the receptors may include, for example, those cases where the *in vitro* activities for the receptors are about 0.001% or higher, about 0.01% or higher, about 0.1% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, about 100% or higher, about 150% or higher, or about 200% or more, compared with native ligands. However, the immunoglobulin Fc region of the present invention is not limited thereto.

The peptide may have an increased *in vivo* half-life compared with any one of native GLP-1, native glucagon, and native GIP, but is not particularly limited thereto.

Although not particularly limited, the peptide may be a non-naturally occurring peptide.

Although described as a peptide "consisting of" a specific SEQ ID NO herein, such description does not exclude a mutation that may occur by the addition of a meaningless sequence upstream or downstream of the amino acid sequence of the corresponding SEQ ID NO, or a mutation that may occur naturally, or a silent mutation thereof, as long as the peptide has an activity identical or corresponding to that of the peptide consisting of the amino acid sequence of the corresponding SEQ ID NO, and it would be obvious that even a peptide with such a sequence addition or mutation falls within the scope of the present invention. In other words, if a peptide shows at least a certain level of homology and exhibits activities for glucagon, GLP-1, and GIP receptors despite having some sequence differences, such a peptide may fall within the scope of the present invention.

The above description can be applied to other embodiments or aspects of the present invention, but is not limited thereto.

For example, reference may be made to WO 2017-116204 and WO 2017-116205 for the peptide of the present invention.

For example, the peptide of the present invention may also include a peptide containing an amino acid sequence of any one of SEQ ID NOS: 1 to 102, consisting (essentially) of any one of amino acid sequences of SEQ ID NOS 1 to 102, or having sequence identity of at least 60%, 65%, 70%, 75%, 80%, 85%, 90%, 91%, 92%, 93%, 94% or 95% with an amino acid sequence of any one of SEQ ID NOS: 1 to 102, and such an amino acid sequence is not limited to specific sequences as long as they have an effect of treating and/or preventing multiple sclerosis.

Examples of the triple agonist may include a triple agonist containing an amino acid sequence selected from the group consisting of SEQ ID NOS: 1 to 102, a triple agonist containing an amino acid sequence selected from the group consisting of SEQ ID NOS: 1 to 11 and 13 to 102, and a triple agonist containing an amino acid sequence selected from the group consisting (essentially) of an amino acid sequence selected from the group consisting of SEQ ID NO: 1 to 11 and 13 to 102, but are not limited thereto.

As a specific example, the triple agonist peptide may contain, consist essentially of, or consist of an amino acid sequence of any one of SEQ ID NOS: 21 to 24, 28, 29, 31, 32, 37, 42, 43, 50, 51 to 54, 56, 58, 64 to 73, 75 to 79, 82, 83, 91, and 96 to 102, but is not limited thereto.

As an example, the triple agonist peptide may contain, consist essentially of, or consist of an amino acid sequence of any one of SEQ ID NOS: 21, 22, 42, 43, 50, 64, 66, 67, 70, 71, 76, 77, 96, 97, and 100, but is not limited thereto.

As another example, the peptide may contain, consist essentially of, or consist of an amino acid sequence of any one of SEQ ID NOS: 21, 22, 42, 43, 50, 66, 67, 77, 96, 97, and 100, but is not limited thereto.

As still another example, the peptide may contain, consist essentially of, or consist of an amino acid sequence of any one of SEQ ID NOS: 21, 22, 42, 43, 50, 77, and 96, but is not limited thereto.

As used herein, the term "homology" or "identity" refers to a degree of relevance between two given amino acid sequences or nucleotide sequences, and the term may be expressed as a percentage.

The terms homology and identity may be often used interchangeably with each other.

The homology, similarity, or identity between any two peptide sequences may be determined by, for example, a known computer algorithm, such as the "FASTA" program, by using default parameters as in Pearson et al (1988) [Proc. Natl. Acad. Sci. USA 85]: 2444. Alternatively, these may be determined using the Needleman-Wunsch algorithm (Needleman and Wunsch, 1970, J. Mol. Biol. 48: 443-453), which is performed in the Needleman program of the European Molecular Biology Open Software Suite (EMBOSS) package (Rice et al., 2000, Trends Genet. 16: 276-277) (version 5.0.0 or versions thereafter) (GCG program package (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the homology, similarity, or identity may be determined using BLAST of the National Center for Biotechnology Information database, or ClustalW.

The homology, similarity, or identity between polypeptides may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines similarity as the number of aligned symbols (i.e., amino acids) which are similar, divided by the total number of symbols in a shorter of two sequences. Default parameters for the GAP program may include: (1) a binary comparison matrix (containing a value of 1 for identity and 0 for non-identity) and a weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps. Therefore, the term "homology" or "identity" used herein represents the relevance between sequences.

The peptide having activities for glucagon, GLP-1, and GIP receptors may include an intramolecular bridge (e.g., a covalent bridge or non-covalent bridge) and, specifically, may be in the form of including a ring, for example, may be in the form of including a ring formed between the amino acids at positions 16 and 20 in the peptide, but the peptide is not particularly limited thereto.

Non-limiting examples of the ring may include a lactam bridge (or a lactam ring).

Additionally, the peptide includes all of those which are modified to include a ring, or to include amino acids capable of forming a ring at a target site.

For example, the pair of the amino acids at positions 16 and 20 in the peptide may be substituted with glutamic acid or lysine, which can form a ring, but the peptide is not limited thereto.

Such a ring may be formed between side chains of amino acids in the peptide, for example, in the form of a lactam ring formed between a side chain of lysine and a side chain of glutamic acid, but is not particularly limited thereto.

Examples of the peptide prepared by a combination of these methods include a peptide, of which the amino acid sequence differs from that of native glucagon in at least one amino acid, from which α-carbon of the amino acid residue at the N-terminus is deleted, and retains activities for glucagon, GLP-1, and GIP receptors, but are not limited thereto. Peptides applicable to the present invention may be prepared by combining various methods for the preparation of analogs.

Additionally, the peptide of the present invention may have the substitution of some amino acids with other amino acids or non-native compounds in order to avoid the recognition by an agonist protease to increase the *in vivo* half-life of the peptide, but is not particularly limited thereto.

Specifically, the peptide of the present invention may be a peptide in which the *in vivo* half-life is increased by avoiding the recognition by a protease through a substitution of the second amino acid in the amino acid sequence of the peptide, but any substitution or change of amino acids to avoid the recognition by an *in vivo* protease is included without limitation.

Such a modification for peptide preparation includes all of: modifications using L-type or D-type amino acids and/or non-native amino acids; and/or modifications of a native sequence, for example, a variation of a side chain functional group, an intramolecular covalent linkage (e.g., ring formation between side chains), methylation, acylation, ubiquitination, phosphorylation, aminohexanation, biotinylation, or the like.

In addition, such a variation also includes all those where one or more amino acids are added to the amino and/or carboxy terminus of native glucagon.

Regarding the substitution or addition of amino acids, not only the 20 amino acids commonly found in human proteins, but also atypical or non-naturally occurring amino acids may be used. Commercial sources of atypical amino acids may include Sigma-Aldrich, ChemPep, and Genzyme Pharmaceuticals. The peptides including these amino acids and typical peptide sequences can be synthesized and purchased from commercial peptide suppliers, for example, American Peptide Company and Bachem in USA, or Anygen in Korea.

Amino acid derivatives may also be accessible in a similar manner, and for example, 4-imidazoacetic acid or the like may be used.

The peptide according to the present invention may be in a varied form in which the N-terminus and/or C-terminus is chemically modified or protected by organic groups, or amino acids are added to the terminus of the peptide, for its protection from proteases *in vivo* while increasing its stability.

In particular, a chemically synthesized peptide has electrically charged N- and C-termini, and thus for elimination of these charges, the N-terminus may be acetylated and/or the C-terminus may be amidated, but the peptide is not particularly limited thereto.

Specifically, the N-terminus or the C-terminus of the peptide of the present invention may have an amine group (-NH2) or a carboxy group (-COOH), but is not limited thereto.

The peptide according to the present invention may include a peptide, of which the C-terminus is amidated or has a free carboxy (-COOH) group, or a peptide, of which the C-terminus is not modified, but is not limited thereto.

In an embodiment, the C-terminus of the peptide may be amidated, but is not limited thereto.

In an embodiment, the peptide may be aglycosylated, but is not limited thereto.

The peptide of the present invention may be synthesized through solid-phase synthesis, produced by a recombinant method, or prepared by a commercial request, but is not limited thereto.

In addition, the peptide of the present invention may be synthesized depending on the length thereof by a method well known in the art, for example, an automatic peptide synthesizer, and may be produced by genetic engineering technology.

Specifically, the peptide of the present invention may be prepared by a standard synthesis method, a recombinant expression system, or any other method known in the art. Hence, the peptide according to the present invention may be synthesized by a number of methods including, for example, the following:
(a) a method in which the peptide is synthesized by means of a solid-phase or liquid-phase method either stepwise or by fragment assembling and a final peptide product is isolated and purified;
(b) a method in which a nucleic acid construct encoding a peptide is expressed in a host cell and an expression product is collected from a host cell culture;
(c) a method in which the *in vitro* cell-free expression of a nucleic acid construct encoding a peptide is performed and an expression product is collected therefrom; or
a method in which peptide fragments are obtained by any combination of (a), (b), and (c), the fragments are linked to obtain a peptide, and then the corresponding peptide is collected.

In addition, the peptide having activities for glucagon, GLP-1, and GIP receptors may be in the form of a long-acting conjugate in which a biocompatible substance for increasing the *in vivo* half-life of a peptide is bound to a peptide having activities for glucagon, GLP-1, and GIP receptors. Herein, the biocompatible substance may be used interchangeably with a carrier. The peptide contained in the pharmaceutical composition of the present invention may be in the form of a long-acting conjugate.

In the present invention, the conjugate of the peptide can exhibit an increase in the duration of efficacy compared with the peptide to which a carrier is not bound, and in the present invention, such a conjugate is referred to as a "long-acting conjugate".

Such a conjugate may be a non-naturally occurring conjugate.

In a specific embodiment of the present invention, the long-acting conjugate refers to a form in which an immunoglobulin Fc region is linked to a peptide having activities for glucagon, GLP-1, and GIP receptors. Specifically, the conjugate may be a conjugate in which an immunoglobulin Fc region is covalently linked to a peptide having activities for glucagon, GLP-1, and GIP receptors via a linker, but is not particularly limited thereto.

In an embodiment of the present invention, the long-acting conjugate may be represented by Formula 1 below, but is not limited thereto:

[Formula 1] X-L-F

wherein X represents a peptide containing an amino sequence of any one of SEQ ID NOS: 1 to 102;
L represents a linker containing ethylene glycol repeat units;
F represents an immunoglobulin Fc region; and
"-" symbols represent covalent linkages between X and L and between L and F, respectively.

Herein, the term "long-acting conjugate of Formula 1" refers to a form in which a peptide including an amino acid sequence of any one of SEQ ID NOS: 1 to 102 is linked to an immunoglobulin Fc region via a linker, and the conjugate may exhibit an increase in the duration of efficacy compared with a peptide including an amino acid sequence of any one of the amino acid sequences of SEQ ID NOS: 1 to 102, to which the immunoglobulin Fc region is not bound.

The conjugate of the present invention, even in the form of a conjugate, can exhibit significant activities for glucagon, GLP-1, and GIP receptors, and thus can also exert an effect of preventing and/or treating multiple sclerosis.

Specifically, the conjugate of the present invention can exhibit *in vitro* activities for glucagon, GLP-1, and/or GIP receptors, of about 0.01% or more, about 0.1% or more, about 0.2% or more, about 0.5% or more, about 0.7% or more, about 1% or more, about 2% or more, about 3 % or more, about 4% or more, about 5% or more, about 6% or more, about 7% or more, about 8% or more, about 9% or more, about 10 % or more, about 20% or more, about 30% or more, about 40% or more, about 50 % or more, about 60% or more, about 70% or more, about 80% or more, about 90% or more, or about 100% or more, compared with native forms, but is not limited thereto.

For the purposes of the present invention, the peptide or the conjugate thereof can exhibit activities for glucagon, GLP-1, and/or GIP receptors, of about 0.01% or more, about 0.1% or more, about 1% or more, about 2% or more, about 3 % or more, about 4% or more, about 5% or more, about 10 % or more, about 20% or more, about 30% or more, about 40% or more, about 50 % or more, about 60% or more, about 70% or more, about 80% or more, about 90 % or more, or about 100% or more, compared with native forms, but is not limited thereto.

The composition of the present invention may contain (i) a peptide having activities for glucagon, GLP-1, and GIP receptors, or (ii) a long-acting conjugate of the peptide having activities for glucagon, GLP-1, and GIP receptors, and the long-acting conjugate can show an excellent effect of preventing and/or treating multiple sclerosis on the basis of an increase in the *in vivo* duration of efficacy.

In the long-acting conjugate, F is a substance capable of increasing the half-life of X, that is, a peptide having activities for glucagon, GLP-1, and GIP receptors, specifically, a peptide containing any one sequence of the amino acid sequences of SEQ ID NO: 1 to 102, and corresponds to one element of a moiety constituting the conjugate of the present invention.

F may be bound to X by a covalent chemical linkage or a non-covalent chemical linkage, and F and X may be linked to each other via a linker by a covalent chemical linkage, a non-covalent chemical linkage, or a combination thereof.

As one example, in the conjugate, X and L and L and F may be linked to each other through covalent linkages, respectively, wherein the conjugate may be a conjugate in which X, L, and F are linked to each other through covalent linkages in the form of Formula 1.

Specifically, the method of linking X, which is a peptide containing an amino acid sequence of any one of SEQ ID NOS: 1 to 102, and an immunoglobulin Fc region in the long-acting conjugate of Formula 1 is not particularly limited, but an amino acid sequence of any one of SEQ ID NOS: 1 to 102 and an immunoglobulin Fc region may be linked to each other via a linker.

Specifically, L may be a non-peptide linker, for example, a linker containing ethylene glycol repeat units.

In the present invention, the "non-peptide linker" includes a biocompatible polymer having two or more repeat units linked to each other. The repeat units are linked to each other by any covalent linkage but not a peptide linkage. The non-peptide linker may be one element constituting a moiety of the conjugate of the present invention, and corresponds to L in Formula 1.

As the non-peptide linker usable in the present invention, any polymer that has resistance to *in vivo* protease may be used without limitation. In the present invention, the non-peptide linker may be used exchangeably with a non-peptide polymer.

In the present invention, the non-peptide linker may include reactive groups at the ends thereof and thus may form a conjugate by reaction with the other elements constituting the conjugate. When the non-peptide linker having reactive functional groups at both ends thereof binds to X and F in Formula 1 through the respective reactive groups to form a conjugate, the non-peptide linker or non-peptide polymer may be named a non-peptide polymer linker moiety or a non-peptide linker moiety.

Although not particularly limited, the non-peptide linker may be a linker containing ethylene glycol repeat units, and for example, may be polyethylene glycol, and derivatives thereof that are already known in the art and derivatives that can be easily prepared within the level of skill in the art also fall within the scope of the present invention.

In the present invention, the "polyethylene glycol linker" includes a biocompatible polymer having two or more repeat units linked to each other. The repeat units are linked to each other by any covalent linkage but not a peptide linkage. The polyethylene glycol linker may be one element constituting a moiety of the conjugate of the present invention, and corresponds to L in Formula 1.

Specifically, L (polyethylene glycol linker) may be a linker containing ethylene glycol repeat units, for example, polyethylene glycol, but is not limited thereto. Herein, the polyethylene glycol is a term encompassing all of the forms of homopolymers of ethylene glycol, PEG copolymers, and monomethyl-substituted PEG polymers (mPEG), but is not particularly limited thereto. In addition, derivatives thereof that are already known in the art and derivatives that can be easily prepared within the skill in the art are also included in the scope of the present invention.

The polyethylene glycol linker may include a functional group used in the preparation of the conjugate before being configured into the conjugate, while including ethylene glycol repeat units. The long-acting conjugate according to the present invention may be in the form in which X and F are linked through the functional group, but is not limited thereto. In the present invention, the non-peptide linker may include two, or three or more functional groups, and each functional group may be the same as or different from each other, but is not limited thereto.

Specifically, the linker may be polyethylene glycol (PEG) represented by Formula 2 below, but is not limited thereto: 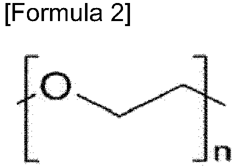 wherein n is 10 to 2400, n is 10 to 480, or n is 50 to 250, but is not limited thereto.

In the long-acting conjugate, the PEG moiety may include not only the (CH₂CH₂O)ₙ structure, but also an oxygen atom interposed between a linking element and the (CH₂CH₂O)ₙ structure, but not limited thereto.

In an embodiment, the ethylene glycol repeat units may be represented by, for example, [OCH₂CH₂]ₙ, wherein the value of n is a natural number, and the average molecular weight, for example, the number average molecular weight of the [OCH₂CH₂]ₙ moiety in the peptide conjugate may be set to more than 0 kDa to about 100 kDa, but is not limited thereto. As another example, the value of n is a natural number, and the average molecular weight, for example, the number average molecular weight of the [OCH2CH2]n moiety in the peptide conjugate may be about 1 to about 100 kDa, about 1 to about 80 kDa, about 1 to about 50 kDa, about 1 to about 30 kDa, about 1 to about 25 kDa, about 1 to about 20 kDa, about 1 to about 15 kDa, about 1 to about 13 kDa, about 1 to about 11 kDa, about 1 to about 10 kDa, about 1 to about 8 kDa, about 1 to about 5 kDa, about 1 to about 3.4 kDa, about 3 to about 30 kDa, about 3 to about 27 kDa, about 3 to about 25 kDa, about 3 to about 22 kDa, about 3 to about 20 kDa, about 3 to about 18 kDa, about 3 to about 16 kDa, about 3 to about 15 kDa, about 3 to about 13 kDa, about 3 to about 11 kDa, about 3 to about 10 kDa, about 3 to about 8 kDa, about 3 to about 5 kDa, about 3 to about 3.4 kDa, about 8 to about 30 kDa, about 8 to about 27 kDa, about 8 to about 25 kDa, about 8 to about 22 kDa, about 8 to about 20 kDa, about 8 to about 18 kDa, about 8 to about 16 kDa, about 8 to about 15 kDa, about 8 to about 13 kDa, about 8 to about 11 kDa, about 8 to about 10 kDa, about 9 to about 15 kDa, about 9 to about 14 kDa, about 9 to about 13 kDa, about 9 to about 12 kDa, about 9 to about 11 kDa, about 9.5 to about 10.5 kDa, or about 10 kDa, but is not limited thereto.

In a specific embodiment, the conjugate may have a structure in which the peptide (X) and the immunoglobulin Fc region (F) are covalently linked via a linker containing ethylene glycol repeat units, but is not limited thereto.

In a specific embodiment, the long-acting conjugate may have a structure in which the peptide (X) and the immunoglobulin Fc region (F) of the present invention are covalently linked via a linker containing ethylene glycol repeat units, but is not limited thereto.

As the non-peptide that can be used in the present invention, any polymer that has a resistance to proteases *in vivo* and contains an ethylene glycol unit may be used without limitation. The molecular weight of the non-peptide polymer may be in a range of more than 0 kDa to about 100 kDa, or about 1 kDa to about 100 kDa, and specifically about 1 kDa to about 20 kDa, or about 1 kDa to about 10 kDa, but is not limited thereto. In addition, as the non-peptide linker of the present invention, which is bound to the polypeptide corresponding to F, not only a single type of polymer but also a combination of different types of polymers may be used. In a specific embodiment, both ends of the non-peptide linker may be linked to a thiol group, an amino group, or a hydroxyl group of the immunoglobulin Fc region and a thiol group, an amino group, an azide group, or a hydroxyl group of the peptide (X), respectively, but are not limited thereto.

Specifically, the linker may include, at both ends thereof, reactive groups capable of binding to F (e.g., the immunoglobulin Fc region) and X, respectively, and specifically reactive groups capable of binding to: a thiol group of cysteine, an amino group located at the N-terminus lysine, arginine, glutamine, and/or histidine, and/or a hydroxyl group located at the C-terminus in the immunoglobulin Fc region; and a thiol group of cysteine; an amino group of lysine, arginine, glutamine, and/or histidine; an azide group of azidolysine; and/or a hydroxyl group in the peptide (X), but is not limited thereto.

Alternatively, the reactive groups of the linker, capable of being linked to F, for example, an immunoglobulin Fc region, and X, may be selected from the group consisting of an aldehyde group, a maleimide group, and a succinimide derivative, but are not limited thereto.

In the above description, examples of the aldehyde group may include a propionaldehyde group or a butyraldehyde group, but are not limited thereto.

In the above description, examples of the succinimide derivative may include succinimidyl valerate, succinimidyl methylbutanoate, succinimidyl methylpropionate, succinimidyl butanoate, succinimidyl propionate, N-hydroxysuccinimide, hydroxy succinimidyl, succinimidyl carboxymethyl, and succinimidyl carbonate, but are not limited thereto.

The linker may be converted into a linker moiety by linking to the immunoglobulin Fc region F and the peptide (triple agonist) X via the reactive groups.

In addition, a final product produced by reductive amination through aldehyde linkage is still more stable than a product obtained through amide linkage. The aldehyde reactive group selectively reacts with the N-terminus at low pH while forming a covalent linkage with a lysine residue at high pH, for example, at pH 9.0.

The reactive groups at both ends of the non-peptide linker may be the same as or different from each other, and for example, one end may have a maleimide group, and the other end may have an aldehyde group, a propionaldehyde group, or a butyraldehyde group. However, the reactive groups are not particularly limited thereto as long as F, specifically an immunoglobulin Fc region, and X can be linked to both ends of the non-peptide linker.

For example, the non-peptide linker may have a maleimide group as a reactive group at one end and an aldehyde group, a propionaldehyde group, a butyraldehyde group, or the like at the other end.

When polyethylene glycol having hydroxyl reactive groups at both ends is used as a non-peptide polymer, the long-acting protein conjugate of the present invention may be prepared by activating the hydroxyl groups into various reactive groups through known chemical reactions or by using commercially available polyethylene glycol having modified reactive groups.

In a specific embodiment, the non-peptide polymer may be linked to a cysteine residue of X, more specifically the -SH group of cysteine, but is not limited thereto.

For example, the non-peptide polymer may be linked to the cysteine residue at position 10, the cysteine residue at position 13, the cysteine residue at position 15, the cysteine residue at position 17, the cysteine residue at position 19, the cysteine residue at position 21, the cysteine residue at position 24, the cysteine residue at position 28, the cysteine residue at position 29, the cysteine residue at position 30, the cysteine residue at position 31, the cysteine residue at position 40, or the cysteine residue at position 41 in the peptide corresponding to X, but is not particularly limited.

Specifically, a reactive group of the non-peptide polymer may be linked to the -SH group of the cysteine residue, and all of those described above are applied to the reactive group. When maleimide-PEG-aldehyde is used, the maleimide group may be linked to the -SH group of X through a thioether linkage, and the aldehyde group may be linked to the -NH₂ group of F, specifically, the immunoglobulin Fc region through reductive amination, but is not limited thereto.

In the conjugate, the reactive group of the non-peptide polymer may be linked to -NH₂ group located at the N-terminus of the immunoglobulin Fc region, but this case corresponds to one example.

When maleimide-PEG-aldehyde is used, the maleimide group may be linked to the -SH group of the peptide through a thioether linkage, and the aldehyde group may be linked to the -NH₂ group of the immunoglobulin Fc region through reductive alkylation, but is not limited thereto, and this case corresponds to one example.

Through such reductive alkylation, the N-terminal amino group of the immunoglobulin Fc region is linked to the oxygen atom located at one terminus of the PEG through a linker functional group having a structure of -CH₂CH₂CH₂- to form a structure of -PEG-O-CH₂CH₂CH₂NH-immunoglobulin Fc, and through a thioether linkage, a structure in which one terminus of PEG is linked to a sulfur atom located at cysteine of the peptide may be formed. The above-described thioether linkage may contain the structure of

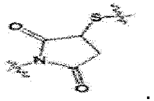

However, the non-peptide polymer is not particularly limited to the above example, and this case corresponds to one example.

In the conjugate, the reactive group of the linker may be linked to -NH₂ located at the N-terminus of the immunoglobulin Fc region, but this is merely an embodiment.

In the conjugate, the peptide according to the present invention may be linked to a linker having a reactive group through the N-terminus thereof, but this is merely an embodiment.

As used herein, the term "C-terminus" refers to the carboxy terminus of the peptide, and for the purpose of the present invention, the term refers to a position that can be linked to a linker. For example, although not limited, the C-terminus may include not only the amino acid residue at the outermost end of the C-terminus but also amino acid residues near the C-terminus, and specifically, the 1st to 20th amino acid residues from the outermost end.

The above-described conjugate may have an increase in the duration of efficacy compared with conjugates having X not modified with F, and such a conjugate includes not only the above-described form but also a form that is encapsulated in a biodegradable nanoparticle.

Meanwhile, F may be an immunoglobulin Fc region and, more specifically, the immunoglobulin Fc region may be derived from IgG, but is not particularly limited thereto.

In a specific embodiment of the present invention, F (the immunoglobulin Fc region) is a dimer consisting of two polypeptide chains and has a structure in which one end of L is linked to only one polypeptide chain of the two polypeptide chains, but is not limited thereto.

In the present invention, the term "immunoglobulin Fc region" refers to a region that includes heavy chain constant region 2 (CH2) and/or heavy chain constant region 3 (CH3) portions, excluding heavy chain and light chain variable regions in an immunoglobulin. The immunoglobulin Fc region may be one element constituting a moiety of the conjugate of the present invention. The immunoglobulin Fc region may be used interchangeably with the term "immunoglobulin Fc fragment".

Herein, the Fc region includes not only native sequences obtained by papain digestion of immunoglobulins, but also derivatives thereof, for example, variants, such as sequences in which one or more amino acid residues in the native sequence have been altered through deletion, insertion, non-conservative or conservative substitution, or a combination of these and is thus different from that of the native form. The derivatives, modifications, and variants are based on the assumption that these retain the ability to bind to FcRn. In the present invention, F may be a human immunoglobulin region, but is not limited thereto. Herein, the "biocompatible substance" or "carrier" may mean the Fc region.

F (immunoglobulin Fc region) has a structure in which two polypeptide chains are linked by a disulfide linkage, with the two chains being linked through only a nitrogen atom of one of the chains, but is not limited thereto. The linking through a nitrogen atom may be performed on the epsilon amino group or the N-terminal amino group of lysine through reductive amination.

The term reductive amination refers to a reaction in which an amine group or an amino group of one reactant reacts with an aldehyde (i.e., a functional group capable of reductive amination) of another reactant to form an amine, which then forms an amine linkage by reductive reaction, and this is an organic synthetic reaction that has been widely known in the art.

In an embodiment, F may be linked through a nitrogen atom of the N-terminus proline thereof, but is not limited thereto.

The immunoglobulin Fc region may be one element constituting a moiety of the conjugate of Formula 1 of the present invention, and may correspond to F in Formula 1.

This immunoglobulin Fc region may include a hinge region in the heavy chain constant region, but is not limited thereto.

In the present invention, the immunoglobulin Fc region may include a specific hinge sequence at the N-terminus.

As used herein, the term "hinge sequence" refers to a site which is located on a heavy chain and forms a dimer of the immunoglobulin Fc region through an inter-disulfide bond.

In the present invention, the hinge sequence may be mutated to have only one cysteine residue by deletion of a part in a hinge sequence having the following amino acid sequence, but is not limited thereto:
Glu-Ser-Lys-Tyr-Gly-Pro-Pro-Cys-Pro-Ser-Cys-Pro (SEQ ID NO: 103).

The hinge sequence may include only one cysteine residue by deletion of the 8th or 11th cysteine residue in the hinge sequence of SEQ ID NO: 103. The hinge sequence of the present invention may consist of 3 to 12 amino acids including only one cysteine residue, but is not limited thereto. More specifically, the hinge sequence of the present invention may have the following sequence: Glu-Ser-Lys-Tyr-Gly-Pro-Pro-Pro-Ser-Cys-Pro (SEQ ID NO: 104), Glu-Ser-Lys-Tyr-Gly-Pro-Pro-Cys-Pro-Ser-Pro (SEQ ID NO: 105), Glu-Ser-Lys-Tyr-Gly-Pro-Pro-Cys-Pro-Ser (SEQ ID NO: 106), Glu-Ser-Lys-Tyr-Gly-Pro-Pro-Cys-Pro-Pro (SEQ ID NO: 107), Lys-Tyr-Gly-Pro-Pro-Cys-Pro-Ser (SEQ ID NO: 108), Glu-Ser-Lys-Tyr-Gly-Pro-Pro-Cys (SEQ ID NO: 109), Glu-Lys-Tyr-Gly-Pro-Pro-Cys (SEQ ID NO: 110), Glu-Ser-Pro-Ser-Cys-Pro (SEQ ID NO: 111), Glu-Pro-Ser-Cys-Pro (SEQ ID NO: 112), Pro-Ser-Cys-Pro (SEQ ID NO: 113), Glu-Ser-Lys-Tyr-Gly-Pro-Pro-Ser-Cys-Pro (SEQ ID NO: 114), Lys-Tyr-Gly-Pro-Pro-Pro-Ser-Cys-Pro (SEQ ID NO: 115), Glu-Ser-Lys-Tyr-Gly-Pro-Ser-Cys-Pro (SEQ ID NO: 116), Glu-Ser-Lys-Tyr-Gly-Pro-Pro-Cys (SEQ ID NO: 117), Lys-Tyr-Gly-Pro-Pro-Cys-Pro (SEQ ID NO: 118), Glu-Ser-Lys-Pro-Ser-Cys-Pro (SEQ ID NO: 119), Glu-Ser-Pro-Ser-Cys-Pro (SEQ ID NO: 120), Glu-Pro-Ser-Cys (SEQ ID NO: 121), Ser-Cys-Pro (SEQ ID NO: 122).

More specifically, the hinge sequence may include an amino acid sequence of SEQ ID NO: 113 (Pro-Ser-Cys-Pro) or SEQ ID NO: 122 (Ser-Cys-Pro), but is not limited thereto.

The immunoglobulin Fc region of the present invention may be in the form in which two molecules of the immunoglobulin Fc chain form a dimer due to the presence of a hinge sequence, and the conjugate of Formula 1 of the present invention may be in the form in which one terminus of the linker is linked to one chain of the dimeric immunoglobulin Fc region, but is not limited thereto.

As used herein, the term "N-terminus" refers to an amino terminus of a protein or polypeptide, and may include the outermost end of the amino terminus, or 1, 2, 3, 4, 5, 6, 7, 8, 9, or 10 or more amino acids from the outermost end. In the immunoglobulin Fc region of the present invention, a hinge sequence may include in the N-terminus, but is not limited thereto.

The immunoglobulin Fc region of the present invention may be an extended Fc region including a part or the entirety of heavy chain constant region 1 (CH1) and/or light chain constant region 1 (CL1), excluding only heavy chain and light chain variable regions of an immunoglobulin, as long as the immunoglobulin Fc region has a substantially equivalent or improved effect compared with the native form. Alternatively, the immunoglobulin Fc region of the present invention may be a region in which a considerably long part of the amino acid sequence corresponding to CH2 and/or CH3 is deleted.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of one or more domains among a CH1 domain, a CH2 domain, a CH3 domain, and a CH4 domain and an immunoglobulin hinge region (or a part of the hinge region); and 6) a dimer of each domain of a heavy chain constant region and a light chain constant region, but is not limited thereto. However, the immunoglobulin Fc region of the present invention is not limited thereto.

In the present invention, the immunoglobulin Fc region may be in the form of a dimer or multi-mer consisting of single-chain immunoglobulins consisting of domains of the same origin, but is not limited thereto.

In an embodiment, the immunoglobulin Fc region may be in a dimeric form, and one molecule of X may be covalently linked to one Fc region in a dimeric form, wherein the immunoglobulin Fc and X may be linked to each other by a non-peptide polymer. In an embodiment of the long-acting conjugate of the present invention, the immunoglobulin Fc region F is a dimer consisting of two polypeptide chains, wherein the dimeric Fc region F and X may be covalently linked to each other via one identical linker L containing ethylene glycol repeat units. In a specific embodiment, X is covalently linked only to one polypeptide chain of the two polypeptide chains of the dimeric Fc region F via the linker L. In a more specific embodiment, only one molecule of X may be covalently linked via L to one polypeptide chain, to which X is linked, of the two polypeptide chains of the dimeric Fc region F. In a most specific embodiment, F is a homodimer.

In another embodiment, the immunoglobulin Fc region F may be a dimer consisting of two polypeptide chains, wherein one end of L is linked only to one polypeptide chain of the two polypeptide chains, but is not limited thereto.

In another embodiment of the long-acting conjugate of the present invention, two molecules of X may be symmetrically bound to one Fc region in a dimeric form. The immunoglobulin Fc and X may be linked to each other via a non-peptide linker. However, the present invention is not limited to the above-described embodiments.

The immunoglobulin Fc region of the present invention includes not only the native amino acid sequence as well as a derivative thereof. The derivative of the native amino acid sequence means an amino acid sequence which is different from the native amino acid sequence by a deletion, an insertion, a non-conservative or conservative substitution, or a combination thereof of one or more amino acid residues in the native amino acid sequence.

For example, the amino acid residues at positions 214 to 238, 297 to 299, 318 to 322, or 327 to 331, which are known to be important for linkage in IgG Fc, may be used as appropriate sites for variation.

In addition, various types of derivatives are possible by, for example, removing a region capable of forming a disulfide bond, deleting some amino acid residues at the N-terminus of native Fc, or adding a methionine residue at the N-terminus of native Fc. In addition, a complement-binding site, for example, a C1q-binding site, may be removed, and an antibody dependent cell mediated cytotoxicity (ADCC) site may be removed to remove effector functions. Techniques for preparing such sequence derivatives of the immunoglobulin Fc region are disclosed in WO 97/34631 and WO 96/32478, and the like.

Amino acid exchanges in proteins and peptides, which do not alter the entire activity of molecules, are well known in the art (H. Neurath, R. L. Hill, The Proteins, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, or the like.

The above-described Fc derivatives exhibit a biological activity equivalent to that of the Fc region of the present invention, and may be obtained by improving the structural stability of the Fc region against heat, pH, and the like.

Such an Fc region may be obtained from native forms isolated from living bodies of humans or animals, such as cows, goats, pigs, mice, rabbits, hamsters, rats, and guinea pigs, or may be recombinant forms or derivatives thereof obtained from transformed animal cells or microorganisms. In particular, the Fc region may be obtained from a native immunoglobulin by isolating a whole immunoglobulin from a living human or animal body and then treating the isolated immunoglobulin with protease. The isolated immunoglobulin is cleaved into Fab and Fc by treatment with papain, and cleaved into pF'c and F(ab)₂ by treatment with pepsin. These may be subjected to size-exclusion chromatography or the like to separate Fc or pF'c therefrom. In a more specific embodiment, the immunoglobulin Fc region is a recombinant immunoglobulin Fc region obtained from microorganisms.

In addition, the immunoglobulin Fc region may have native glycans, increased or decreased glycans compared with the native form, or be in a deglycosylated form. The increase, decrease, or removal of the immunoglobulin Fc glycans may be achieved by using conventional methods, such as a chemical method, an enzymatic method, and a genetic engineering method using a microorganism. The immunoglobulin Fc region obtained by removal of glycans from Fc shows a significant deterioration in binding affinity to the complement c1q and a decrease or elimination in antibody-dependent cytotoxicity or complement-dependent cytotoxicity, and thus cause no unnecessary immune response *in vivo.* In this regard, a deglycosylated or aglycosylated immunoglobulin Fc region may be a more suitable form to meet the original purpose of the present invention as a drug carrier.

As used herein, the term "deglycosylation" means the enzymatic removal of sugars from an Fc region, while the term "aglycosylation" means an unglycosylated Fc region produced in prokaryotes, more specifically, *E. coli.*

Meanwhile, the immunoglobulin Fc region may be originated from humans, or other animals including cows, goats, pigs, mice, rabbits, hamsters, rats, and guinea pigs, and in a more specific embodiment, the immunoglobulin Fc region is originated from humans.

In addition, the immunoglobulin Fc region may be an Fc region derived from IgG, IgA, IgD, IgE, IgM, or a combination or hybrid thereof. In a still more specific embodiment, the immunoglobulin Fc region is derived from IgG or IgM, which is most abundant in the human blood, and in a still more specific embodiment, the immunoglobulin Fc region is derived from IgG, which is known to increase the half-lives of ligand-binding proteins. In a still more specific embodiment, the immunoglobulin Fc region is an IgG4 Fc region, and in a most specific embodiment, the immunoglobulin Fc region is an aglycosylated Fc region derived from human IgG4, but is not limited thereto.

In a specific embodiment, the immunoglobulin Fc region, which is a fragment of human IgG4 Fc, may be in the form of a homodimer in which two monomers are linked through a disulfide bond (inter-chain form) between cysteines, which are the third amino acids of the monomers, respectively. In particular, each monomer of the homodimer independently have/may have an inter-disulfide bond between cysteines at positions 35 and 95 and an inter-disulfide bond between cysteines at positions 141 and 199, that is, two inter-disulfide bonds (intra-chain form). With respect to the number of amino acids, each monomer may consist of 221 amino acids, and the number of the amino acids forming the homodimer may be a total of 442, but the number of amino acids is not limited thereto. Specifically, in the immunoglobulin Fc fragment, two monomers having the amino acid sequence of SEQ ID NO: 123 (consisting of 221 amino acids) form a homodimer through an inter-disulfide bond between cysteines, which are the 3rd amino acid of each monomer, wherein the monomers of the homodimer independently form an inter-disulfide bond between the cysteines at positions 35 and 95 and an inter-disulfide bond between the cysteines at positions 141 and 199, respectively, but the immunoglobulin Fc fragment is not limited thereto.

F in Formula 1 may include a monomer having the amino acid sequence of SEQ ID NO: 123, wherein F may be a homodimer of monomers having the amino acid sequence of SEQ ID NO: 123, but is not limited thereto.

As one example, the immunoglobulin Fc region may be a homodimer including the amino acid sequence of SEQ ID NO: 124 (consisting of 442 amino acids), but is not limited thereto.

In an embodiment, the immunoglobulin Fc region and X may not be glycosylated, but are not limited thereto.

As used herein, the term "combination" means that when a dimer or multimer is formed, a polypeptide encoding the single-chain immunoglobulin constant region of the same origin (specifically an Fc region) forms a bond with a single-chain polypeptide of different origin. That is, a dimer or multimer may be prepared from two or more fragments selected from the group consisting of IgG Fc, IgA Fc, IgM Fc, IgD Fc, and IgE Fc fragments.

As used herein, the term "hybrid" means that a sequence corresponding to two or more immunoglobulin Fc fragments of different origins is present in a single-chain immunoglobulin constant region. In the present invention, several types of hybrid are possible. That is, the hybrid domain may be composed of one to four domains selected from the group consisting of CH1, CH2, CH3, and CH4 of IgG Fc, IgM Fc, IgA Fc, IgE Fc, and IgD Fc can be hybridized, and may include a hinge region.

Meanwhile, IgG may also be divided into IgG1, IgG2, IgG3, and IgG4 subclasses, and the present invention may also include a combination thereof or a hybridization thereof. Specifically, IgG may be IgG2 and IgG4 subclasses, and more specifically, an Fc fragment of IgG4 rarely having effector functions, such as complement dependent cytotoxicity (CDC).

The above-described conjugate may have an increase in the duration of efficacy compared with native GLP-1, GIP, or glucagon or with X which is not modified with F, and such a conjugate includes not only the above-described form but also a form that is encapsulated in a biodegradable nanoparticle, but is not limited thereto.

The above-described conjugate may have an increase in the duration of efficacy compared with native GLP-1, GIP, or glucagon or with X which is not modified with F, and such a conjugate includes not only the above-described form but also a form that is encapsulated in a biodegradable nanoparticle.

With respect to the above-described triple agonist and the long-acting conjugate thereof, the entire contents of International Patent Publication Nos. WO 2017/116204 and WO 2017/116205 are incorporated herein by reference.

Unless specified otherwise herein, the contents in the detailed description and claims with respect to the "peptide" according to the present invention or a "conjugate" in which this peptide is covalently linked to a biocompatible substance through a covalent linkage are applied to the ranges including all of not only the corresponding peptide or conjugate but also salts of the corresponding peptide or conjugate (e.g., pharmaceutically acceptable salts of the peptide) or solvates thereof. Therefore, although described as only "peptide" or "conjugate" herein, the corresponding described contents are equally applied to a specific salt thereof, a specific solvate thereof, and a specific solvate of the specific salt thereof. These salts may be in the form in which, for example, any pharmaceutically acceptable salt is used. The type of the salt is not particularly limited. However, the salt is preferably in the form that is safe and effective to a subject, e.g., a mammal, but is not particularly limited thereto.

The type of the salt is not particularly limited. However, the salt is preferably in the form that is safe and effective to a subject, e.g., a mammal, but is not particularly limited thereto.

The term "pharmaceutically acceptable" refers to a substance that can be effectively used for a desired purpose without causing excessive toxicity, irritation, allergic responses, and the like within the range of the medical and pharmaceutical decision.

As used herein, the term "pharmaceutically acceptable salt" refers to a salt derived from pharmaceutically acceptable inorganic acids, organic acids, or bases. Examples of appropriate acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Salts derived from appropriate bases may include alkali metals such as sodium and potassium, alkali earth metals such as magnesium, ammonium, and the like.

As used herein, the term "solvate" refers to a complex formed between the peptide according to the present invention or a salt thereof and a solvent molecule.

A composition containing the peptide (e.g., the peptide itself or a long-acting conjugate form in which a biocompatible substance is bound to the peptide) may be used for the prevention or treatment of multiple sclerosis.

The composition according to the present invention may contain a peptide (e.g., the peptide itself or a long-acting conjugate form in which a biocompatible substance is bound to the peptide), and specifically may contain a pharmaceutically effective amount of the peptide or a long-acting conjugate thereof. The composition of the present invention may further contain a pharmaceutically acceptable carrier.

As used herein, the term "prevention" refers to any action that inhibit or delay the occurrence of multiple sclerosis by administration of the above peptide (e.g., the peptide itself or a long-acting conjugate form in which a biocompatible substance is bound to the peptide) or a composition containing the peptide, while the term "treatment" refers to any action that alleviates or advantageously change the symptoms of multiple sclerosis by administration of the above peptide (e.g., the peptide itself or a long-acting conjugate form in which a biocompatible substance is bound to the peptide) or a composition containing the peptide.

As used herein, the term "administration" refers to the introduction of a predetermined substance into a patient by any appropriate method, and the route of administration of the composition may include, but is not particularly limited to, any typical route th rough which the composition can arrive at a target *in vivo,* and for example, the route of administration may be intraperitoneal administration, intravenous administration, intramuscular administration, subcutaneous administration, intradermal administration, oral administration, topical administration, intranasal administration, intrapulmonary administration, intrarectal administration, or the like.

The use of the triple agonist and the long-acting conjugate thereof that has activities for all of long-acting glucagon, GLP-1, and GIP receptors can show a remarkable increase in the blood half-life and *in vivo* duration of efficacy, thereby reducing the frequency of administration to chronic patients suffering from daily injections, leading to an improvement in the quality of life in the patients, and thus the triple agonist and the long-acting conjugate thereof of the present invention helps in the treatment of multiple sclerosis. Furthermore, the triple agonist or a long-acting conjugate thereof greatly helps in the prevention and/or treatment of multiple sclerosis, such as preventing multiple sclerosis and/or significantly reducing the symptoms of multiple sclerosis, by delaying the relapse of the multiple sclerosis.

As used herein, the term "multiple sclerosis (MS)" is a the most common type of demyelinating disease (a disease in which myelin sheath, an insulating material surrounding the axon of neurons, is demylelinated) in the central nervous system and is a chronic inflammatory disease that mainly occurs in the younger age group. The myelin sheath is an insulating substance that surrounds neuronal axons, and the peeling off and demylineation of myelin sheath causes abnormalities in the conduction of neuronal signals, resulting in the death of neurons. Multiple sclerosis is clinically characterized by repeated relapses and remissions, and in the initial stage, symptoms are alleviated without disability after a relapse, but as time passes and relapses repeat, the symptoms are not completely alleviated and disability remains. The beginning of treatment in the early stages of the disease can lead to better outcomes than natural healing. Multiple sclerosis may occur in people of all ages, but most cases occur between the ages of 20 and 40 years and rare cases occur before the age of 10 years or after the age of 60 years. Multiple sclerosis occurs twice as often in women than in men.

Multiple sclerosis is characterized by repeated relapses and pathological changes scattered in several sites of the central nervous system. In other words, there are no specific symptoms unique to multiple sclerosis, and various symptoms and signs of the disease are shown according to the occurrence site of pathological symptom.

Frequently, multiple sclerosis shows an acute or subacute symptoms, which are then gradually alleviated. The most common symptoms are sensory symptoms (initial symptoms) and motor disturbance. Sensory symptoms are shown in the form of abnormal sensations, such as numbness, tingling, and burning. Motor disturbance may be shown in various forms, such as hemiplegia, paraplegia, or limb paralysis, according to occurrence site of pathological symptom, but it is not limited thereto. Symptoms of multiple sclerosis may be categorized into primary, secondary, and tertiary symptoms. Specifically, the primary symptoms result directly from demyelination. This impairs the transduction of electrical signals to muscles (which allow proper movement) and body organs (which allow normal functioning). This causes weakness, tremors, tingling, numbness, loss of balance, vision impairment, paralysis, and bladder and bowel problems. The secondary symptoms result from the primary symptoms. For example, paralysis (primary symptom) may be followed by bedsores (pressure sores) and urinary incontinence problems that may cause bladder or urinary tract infections. Tertiary symptoms mean social, psychological, and occupational complications associated with primary and secondary symptoms. For example, depression may occur in people with multiple sclerosis.

An example of the "multiple sclerosis" may be relapsing/remitting multiple sclerosis (RRMS), secondary progressive multiple sclerosis (SPMS), primary progressive multiple sclerosis (PPMS), or progressive relapsing multiple sclerosis (PRMS), but is not limited thereto.

Multiple sclerosis may progress very variously, and the types of multiple sclerosis may be classified according to the progression thereof. Specifically, relapsing/remitting multiple sclerosis (RRMS) is characterized by repeated exacerbations and remissions of symptoms. RRMS is the most common type of multiple sclerosis and occurs early in the progression of multiple sclerosis.

Secondary progressive multiple sclerosis (SPMS) initially begins as the relapsing/remitting multiple sclerosis, but later progresses into a progressive disease. Approximately 50% of patients with relapsing/remitting multiple sclerosis progress to secondary progressive multiple sclerosis within 10 years, and up to 90% of patients convert to secondary progressive multiple sclerosis after 25 years or more.

Primary progressive multiple sclerosis (PPMS) is characterized by steady progression without overt relapse or recovery. Primary progressive multiple sclerosis occurs in 15% of people with multiple sclerosis but is more common in people who develop multiple sclerosis after age 40.

Progressive relapsing multiple sclerosis (PRMS) occurs in 6% to 10% of patients with multiple sclerosis and progresses steadily. Patients with the disease may experience complete or partial recovery after sudden relapses, and unlike relapsing/remitting multiple sclerosis (RRMS), the disease progresses between relapsing phases.

The triple agonist having activities for all of glucagon, GLP-1, and GIP receptors or a long-acting conjugate thereof according to the present invention provides an effect of alleviating and/or delaying paralysis as a multiple sclerosis symptom, and/or an effect of alleviating and/or delaying a symptom in which normal righting reflex is not performed due to multiple sclerosis, but is not limited thereto. The triple agonist having activities for all of glucagon, GLP-1, and GIP receptors or a long-acting conjugate thereof can provide a disease-altering effect in multiple sclerosis. In particular, the administration of the triple agonist having activities for all of glucagon, GLP-1, and GIP receptors or a long-acting conjugate thereof can improve the quality of life by slowing and/or delaying the progression of multiple sclerosis and thus is suitable for the treatment in an initial stage of multiple sclerosis, but is not limited thereto. In addition, the administration of the triple agonist or a long-acting conjugate thereof can significantly reduce symptoms of multiple sclerosis even in a stage of multiple sclerosis where the onset has already progressed to some extent, and thus is suitable for the treatment in the middle/late stages of multiple sclerosis.

The pharmaceutical composition of the present invention may further contain a pharmaceutically acceptable carrier, vehicle, or diluent. The pharmaceutically acceptable carrier, vehicle, or diluent may be non-naturally occurring.

As used herein, the term "pharmaceutically acceptable" refers to the amount enough to show therapeutic effects, without causing side effects, and such an amount can be easily determined by a person skilled in the art depending the factors well-known in a medical field, such as the type of disease, patient's age, weight, health, and sex, patient's sensitivity to drugs, route of administration, method of administration, frequency of administration, duration of treatment, drugs used in combination or concurrently.

The pharmaceutical composition containing the peptide of the present invention may further contain a pharmaceutically acceptable vehicle. With respect to the vehicle, although not particularly limited, but a binder, a lubricant, a disintegrant, a solubilizer, a dispersant, a stabilizer, a suspending agent, a coloring agent, a flavoring agent, and the like may be used for oral administration; a buffer, a preservative, an analgesic, a solubilizer, an isotonic agent, a stabilizer, and the like may be mixed for an injection; and a base, a vehicle, a lubricant, a preservative, and the like may be used for topical administration.

The formulation of the composition of the present invention may be prepared in various manners by mixing with the above-described pharmaceutically acceptable vehicle. For example, for oral administration, the composition of the present invention may be formulated in the form of a tablet, a troche, a capsule, an elixir, a suspension, a syrup, a wafer, or the like; for the injection, the composition may be formulated as a single dose ampoule or a multidose form. The composition may be formulated into a solution, a suspension, a tablet, a pill, a capsule, a long-acting preparation, or the like.

Meanwhile, examples of the carrier, vehicle, and diluent suitable for preparation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oils, and the like. In addition, the composition may further contain a filler, an anti-coagulant, a lubricant, a humectant, a flavoring agent, a preservative, and the like.

In addition, the pharmaceutical composition of the present invention may have any one formulation selected from the group consisting of a tablet, a pill, a powder, granules, a capsule, a suspension, a liquid medicine for internal use, an emulsion, a syrup, a sterile aqueous solution, a non-aqueous solvent, a lyophilized preparation, and a suppository.

The composition may be formulated into a single dose form suitable for the patient's body, and specifically, may be formulated into a preparation useful for the administration of a protein drug, and may be administered by an administration method commonly used in the art through an oral or parenteral route, such as through a skin, intravenous, intramuscular, intra-arterial, intramedullary, intrathecal, intraventricular, pulmonary, transdermal, subcutaneous, intraperitoneal, intranasal, intragastric, topical, sublingual, vaginal, or rectal route, but is not limited thereto.

In addition, the conjugate may be used by mixing with various carriers approved as drugs, such as physiological saline or organic solvents, and for increasing stability or absorptivity, carbohydrates such as glucose, sucrose, or dextran, antioxidants such as ascorbic acid or glutathione, chelating agents, low-molecular weight proteins, or other stabilizers may be used as medical agents.

The dose and frequency of the pharmaceutical composition of the present invention are determined according to the type of drug as an active ingredient, together with various factors, such as a disease to be treated, a route of administration, patient's age, gender, and body weight, and severity of a disease. Specifically, the composition of the present invention may contain a pharmaceutically effective amount of the peptide or the long-acting conjugate containing the peptide, but is not limited thereto.

Containing the peptide or the long-acting conjugate in a pharmaceutically effective amount means a level at which desired pharmaceutical activity (e.g., prevention, alleviation, or treatment of multiple sclerosis) can be obtained by the peptide or a long-acting conjugate thereof, and may also mean a level at which toxicities or side effects are absent or present at a slight level as a pharmaceutically acceptable level in a subject to be administered, but the level is not limited thereto. Such a pharmaceutically effective amount may be determined by comprehensively considering the frequency of administration, patient, formulation, and the like.

Although not particularly limited, the pharmaceutical composition of the present invention may contain the ingredient (active ingredient) in an amount of 0.01 to 99% (w/v).

The total effective amount of the composition of the present invention may be administered to a patient in a single dose, or may be administered in multiple doses by a fractionated treatment protocol for a long period of time. The pharmaceutical composition of the present invention may an active ingredient, of which the content may vary depending on the severity of a disease. Specifically, the preferable total dose of the triple agonist or a long-acting conjugate thereof of the present invention may be about 0.0001 to 500 mg per 1 kg of body weight of a patient per day. However, as for the dose of the triple agonist or a long-acting conjugate thereof, the effective does thereof for a patient is determined considering various factors including patient's age, body weight, health condition, and sex, the severity of a disease, a diet, and a rate of excretion, in addition to the route of administration and the frequency of treatment of the pharmaceutical composition, and thus, considering these, a person skilled in the art may easily determine an appropriate effective dose according to the particular use of the composition of the present invention. The pharmaceutical composition according to the present invention is not particularly limited to the formulation, route of administration, and method of administration, as long as the pharmaceutical composition exhibits the effects of the present invention.

The pharmaceutical composition of the present invention has excellent *in vivo* duration of efficacy and potency, thereby significantly reducing the number and frequency of administration of the pharmaceutical preparation of the present invention.

In accordance with another aspect of the present invention, there is provided a method for preventing or treating multiple sclerosis, the method including administering to a subject in need thereof the triple agonist (peptide) or a long-acting conjugate of the triple agonist or a composition containing the peptide or the long-acting conjugate.

The triple agonist and/or a long-acting conjugate of the triple agonist, or the composition containing the same, the multiple sclerosis, and prevention and treatment thereof are as described above.

The subject refers to a subject suspected of having multiple sclerosis, and the subject suspected of having multiple sclerosis indicates mammals including humans, rats, livestock, and the like, which have or are at the risk of developing the corresponding disease, but any subject that can be treated with the peptide and/or conjugate or the composition containing the same is included without limitation.

As used herein, the term "administration" refers to the introduction of a predetermined substance into a patient by any appropriate method, and the route of administration of the composition may include, but is not particularly limited to, any typical route through which the composition can arrive at a target *in vivo,* and for example, the route of administration may be intraperitoneal administration, intravenous administration, intramuscular administration, subcutaneous administration, intradermal administration, oral administration, topical administration, intranasal administration, intrapulmonary administration, intrarectal administration, or the like.

The method of the present invention may include administering a pharmaceutically effective amount of the pharmaceutical composition containing the peptide or the conjugate thereof. The appropriate total daily dose of the pharmaceutical composition may be determined by a practitioner within the scope of correct medical decision, and the pharmaceutical composition may be administered once or several times in divided doses. For the purpose of the present invention, the specific therapeutically effective dose for a specific patient may be preferably applied differently, depending on various factors well known in the medical art, including the type and degree of response to be achieved, a specific composition including whether other agents are occasionally used therewith or not, the patient's age, body weight, general health condition, sex and a diet, the time of administration, the route of administration, the secretion rate of the composition, the duration of treatment, other drugs used in combination or concurrently with the composition of the present invention, and like factors well-known in the medical art.

In accordance with another aspect of the present invention, there is provided use of a composition containing the triple agonist peptide or a long-acting conjugate of the peptide in the preparation of a medicine for prevention or treatment of multiple sclerosis.

The peptide and/or conjugate, the composition containing the same, multiple sclerosis, and the prevention and treatment thereof are as described above.

In accordance with another aspect of the present invention, there is provided use of the peptide and/or conjugate, or a composition containing the same for preventing or treating multiple sclerosis.

The peptide or the composition containing the same, multiple sclerosis, and the prevention and treatment thereof are as described above.

Hereinafter, the present invention will be described in detail with reference to the following examples. However, the following examples are merely for illustrating the present invention and are not intended to limit the scope of the present invention.

### Example 1: Preparation of triple agonists

Triple agonists showing activities for all of GLP-1, GIP, and glucagon receptors were prepared, and amino acid sequences thereof are shown in Table 1 below.

**TABLE 1**

| SEQ ID NO | Sequence | Information |
|---|---|---|
| 1 | H X Q G T F T S D V S S Y L D G Q A A K E F I A W L V K G C | |
| 2 | H X Q G T F T S D V S S Y L D G Q A Q K E F I A W L V K G C | |
| 3 | HXQGTFTSDVSSYLLGQAAKQFIAWLVKG GGPSSGAPPPSC | |
| 4 | H X Q G T F T S D V S S Y L L G Q Q Q K E F I A W L V K G C | |
| 5 | H X Q G T F T S D V S S Y L L G Q Q Q K E F I A W L V K G G G P S S G A P P P S C | |
| 6 | H X Q G T F T S D V S S Y L D G Q A A K E F V A W L L K G C | |
| 7 | H X Q G T F T S D V S K Y L D G Q A A K E F V A W L L K G C | |
| 8 | H X Q G T F T S D V S K Y L D G Q A A Q E F V A W L L K G C | |
| 9 | H X Q G T F T S D V S K Y L D G Q A A Q E F V A W L L A G C | |
| 10 | H X Q G T F T S D V S K Y L D G Q A A Q E F V A W L L A G G G P S S G A P P P S C | |
| 11 | CAGEGTFTSDLSKYLDSRRQQLFVQWLKA GGPSSGAPPPSHG | |
| 12 | CAGEGTFISDLSKYMDEQAVQLFVEWLMA GGPSSGAPPPSHG | |
| 13 | CAGEGTFISDYSIQLDEIAVQDFVEWLLAQ KPSSGAPPPSHG | |
| 14 | CAG Q G T F T S D Y S I Q L D E I A V R D F V E W L K N G G P S S G A P P P S H G | |
| 15 | CAG Q G T F T S D L S K Q M D E E A V R L F I E W L K N G G P S S G A P P P S H G | |
| 16 | CA G Q G T F T S D L S K Q M D S E A Q Q L F I E W L K N G G P S S G A P P P S H G | |
| 17 | CAGQGTFTSDLSKQMDEERAREFIEWLLA QKPSSGAPPPSHG | |
| 18 | CAGQGTFTSDLSKQMDSERAREFIEWLKN TGPSSGAPPPSHG | |
| 19 | CAGQGTFTSDLSIQYDSEHQRDFIEWLKD TGPSSGAPPPSHG | |
| 20 | CA G Q G T F T S D L S I Q Y E E E A Q Q D F V E W L K D T G P S S G A P P P S H G | |
| 21 | Y X Q G T F T S D Y S K Y L D E C R A K E F V Q W L L D H H P S S G Q P P P S | Ring formation |
| 22 | Y X Q G T F T S D Y S K C L D E K R A K E F V Q W L L D H H P S S G Q P P P S | Ring formation |
| 23 | Y X Q G T F T S D Y S K Y L D E C R A K E F V Q W L L A Q K G K K N D W K H N I T | Ring formation |
| 24 | Y X Q G T F T S D Y S K Y L D E C R A K E F V Q W L K N G G P S S G A P P P S | Ring formation |
| 25 | H X Q G T F T S D C S K Y L D E R A A Q D F V Q W L L D G G P S S G A P P P S | |
| 26 | H X Q G T F T S D C S K Y L D S R A A Q D F V Q W L L D G G P S S G A P P P S | |
| 27 | H X Q G T F T S D Y S K Y L D E R A C Q D F V Q W L L D Q G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D E K A A K E F V Q W L L N T C | Ring formation |
| 30 | H X Q G T F T S D Y S K Y L D E K A Q K E F V Q W L L D T C | Ring formation |
| 31 | X Q G T F T S D Y S K Y L D E K A C K E F V Q W L L A Q | Ring formation |
| 32 | H X Q G T F T S D Y S K Y L D E K A C K D F V Q W L L D G G P S S G A P P P S | Ring formation |
| 33 | H X Q G T F T S D Y S I A M D E I H Q K D F V N W L L A Q K C | Ring formation |
| 34 | H X Q G T F T S D Y S K Y L D E K R Q K E F V N W L L A Q K C | Ring formation |
| 35 | H X Q G T F T S D Y S I A M D E I H Q K D F V N W L L N T K C | Ring formation |
| 36 | HXQGTFTSDYSKYLCEKRQKEFVQWLLN GGPSSGAPPPSG | Ring formation |
| 37 | H X Q G T F T S D Y S K Y L D E C R Q K E F V Q W L L N G G P S S G A P P P S G | Ring formation |
| 38 | CAXQGTFTSDKSSYLDERAAQDFVQWLLD GGPSSGAPPPSS | |
| 39 | HXQGTFTSDYSKYLDGQHAQCFVAWLLAG GGPSSGAPPPS | |
| 40 | HXQGTFTSDKSKYLDERACQDFVQWLLD GGPSSGAPPPS | |
| 41 | HXQGTFTSDKSKYLDECAAQDFVQWLLDG GPSSGAPPPS | |
| 42 | Y X Q G T F T S D Y S K Y L D E K R A K E F V Q W L L D H H P S S G Q P P P S C | Ring formation |
| 43 | Y X Q G T F T S D Y S K Y L D E K R A K E F V Q W L L D H H C S S G Q P P P S | Ring formation |
| 44 | HGQGTFTSDCSKQLDGQAAQEFVAWLLA GGPSSGAPPPS | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | H G Q G T F T S D C S K Y L D G Q R A Q E F V A W L L A G G P S S G A P P P S | |
| 48 | H G Q G T F T S D C S K Y L D G Q R A Q D F V N W L L A G G P S S G A P P P S | |
| 49 | C A X Q G T F T S D Y S I C M D E I H Q K D F V N W L L N T K | Ring formation |
| 50 | H X Q G T F T S D Y S K Y L D E K R A K E F V Q W L L D H H P S S G Q P P P S C | Ring formation |
| 51 | H X Q G T F T S D Y S K Y L D E K R Q K E F V Q W L L N T C | Ring formation |
| 52 | H X Q G T F T S D Y S K Y L D E K R Q K E F V Q W L L D T C | Ring formation |
| 53 | H X E G T F T S D Y S I A M D E I H Q K D F V N W L L A Q C | Ring formation |
| 54 | H X E G T F T S D Y S I A M D E I H Q K D F V D W L L A E C | Ring formation |
| 55 | H X Q G T F T S D Y S I A M D E I H Q K D F V N W L L A Q C | Ring formation |
| 56 | H X Q G T F T S D Y S K Y L D E K R Q K E F V N W L L A Q C | Ring formation |
| 57 | H X Q G T F T S D Y S I A M D E I H Q K D F V N W L L N T C | Ring formation |
| 58 | H X Q G T F T S D Y S K Y L D E K R Q K E F V Q W L L N T K C | Ring formation |
| 59 | CAXQGTFTSDYSICMDEKHQKDFVNWLLN TK | Ring formation |
| 60 | CAX Q G T F T S D Y S I A M D E K H C K D F V N W L L N T K | Ring formation |
| 61 | CAX Q G T F T S D Y S I A M D E I A C K D F V N W L L N T K | Ring formation |
| 62 | CAXQGTFTSDKSKYLDERAAQDFVQWLLD GGPSSGAPPPS | |
| 63 | CAXQGTFTSDCSKYLDERAAQDFVQWLLD GGPSSGAPPPS | |
| 64 | Y X Q G T F T S D Y S K Y L D E C A A K E F V Q W L L D H H P S S G Q P P P S | Ring formation |
| 65 | H X Q G T F T S D Y S K C L D E K R A K E F V Q W L L D H H P S S G Q P P P S | Ring formation |
| 66 | Y X Q G T F T S D Y S K Y L D E C R A K D F V Q W L L D H H P S S G Q P P P S | Ring formation |
| 67 | Y X Q G T F T S D Y S K Y L D E C A A K D F V Q W L L D H H P S S G Q P P P S | Ring formation |
| 68 | Y X Q G T F T S D Y S K C L D E K A A K E F V Q W L L D H H P S S G Q P P P S | Ring formation |
| 69 | Y X Q G T F T S D Y S K C L D E R A A K E F V Q W L L D H H P S S G Q P P P S | Ring formation |
| 70 | Y X Q G T F T S D Y S K C L D E K R A K D F V Q W L L D H H P S S G Q P P P S | Ring formation |
| 71 | Y X Q G T F T S D Y S K Y L D E R A C K D F V Q W L L D H H P S S G Q P P P S | Ring formation |
| 72 | Y X Q G T F T S D C S K Y L D E R A A K D F V Q W L L D H H P S S G Q P P P S | Ring formation |
| 73 | CAXQGTFTSDYSKYLDECRAKEFVQWLLD HHPSSGQPPPS | Ring formation |
| 74 | CAXQGTFTSDYSKCLDEKRAKEFVQWLLD HHPSSGQPPPS | Ring formation |
| 75 | Y X Q G T F T S D Y S K Y L D E K A A K E F V Q W L L D H H P S S G Q P P P S C | Ring formation |
| 76 | Y X Q G T F T S D Y S K Y L D E K R A K D F V Q W L L D H H P S S G Q P P P S C | Ring formation |
| 77 | Y X Q G T F T S D Y S K Y L D E K A A K D F V Q W L L D H H P S S G Q P P P S C | Ring formation |
| 78 | H X Q G T F T S D Y S K Y L D E K R Q K E F V Q W L L D T K C | Ring formation |
| 79 | H X E G T F T S D Y S I A M D E I H Q K D F V N W L L A Q K C | Ring formation |
| 80 | H X E G T F T S D Y S I A M D E I H Q K D F V D W L L A E K C | Ring formation |
| 81 | CA X Q G T F T S D Y S K Y L D E K R Q K E F V Q W L L N T C | Ring formation |
| 82 | CAXQGTFTSDYSKYLDEKRQKEFVQWLLD TC | Ring formation |
| 83 | CAXEGTFTSDYSIAMDEIHQKDFVNWLLAQ C | Ring formation |
| 84 | CAX E G T F T S D Y S I A M D E I H Q K D F V D W L L A E C | Ring formation |
| 85 | CA X Q G T F T S D Y S I A M D E I H Q K D F V N W L L A Q C | Ring formation |
| 86 | CA X Q G T F T S D Y S K Y L D E K R Q K E F V N W L L A Q C | Ring formation |
| 87 | CAX Q G T F T S D Y S I A M D E I H Q K D F V N W L L N T C | Ring formation |
| 88 | CAXQGTFTSDYSKYLDEKRQKEFVQWLLN TKC | Ring formation |
| 89 | CAXQGTFTSDYSKYLDEKRQKEFVQWLLD TKC | Ring formation |
| 90 | CAXEGTFTSDYSIAMDEIHQKDFVNWLLAQ KC | Ring formation |
| 91 | CAXEGTFTSDYSIAMDEIHQKDFVDWLLAE KC | Ring formation |
| 92 | CAXQGTFTSDYSIAMDEIHQKDFVNWLLA QKC | Ring formation |
| 93 | CAXQGTFTSDYSKYLDEKRQKEFVNWLLA QKC | Ring formation |
| 94 | CAX Q G T F T S D Y S I A M D E I H Q K D F V N W L L N T K C | Ring formation |
| 95 | Y X Q G T F T S D Y S K Y L D E K R A K E F V Q W L L C H H P S S G Q P P P S | Ring formation |
| 96 | YXQGTFTSDYSKYLDEKRAKEFVQWLLDH CPSSGQPPPS | Ring formation |
| 97 | Y X Q G T F T S D Y S K Y L D E K R A K E F V Q W L L D C H P S S G Q P P P S | Ring formation |
| 98 | Y X Q G T F T S D Y S K A L D E K A A K E F V N W L L D H H P S S G Q P P P S C | Ring formation |
| 99 | Y X Q G T F T S D Y S K A L D E K A A K D F V N W L L D H H P S S G Q P P P S C | Ring formation |
| 100 | Y X Q G T F T S D Y S K A L D E K A A K E F V Q W L L D Q H P S S G Q P P P S C | Ring formation |
| 101 | Y X Q G T F T S D Y S K A L D E K A A K E F V N W L L D Q H P S S G Q P P P S C | Ring formation |
| 102 | YXQGTFTSDYSKALDEKAAKDFVNWLLDQ HPSSGQPPPSC | Ring formation |

In the sequences shown in Table 1, the amino acid marked with X represents 2-aminoisobutyric acid (Aib), which is a non-native amino acid, and the underlined amino acids represent amino acids forming a ring together. In Table 1, CA represents 4-imidazoacetyl. The triple agonist peptides were used as triple agonist having the amidated C-terminus, as needed.

### Example 2: Preparation of long-acting conjugates of triple agonists

To PEGylate cysteine residues of the triple agonists (SEQ ID NOS: 21, 22, 42, 43, 50, 77, and 96) of Example 1 with PEG (10 kDa) with a maleimide group and an aldehyde group at both ends, respectively, that is, maleimide-PEG-aldehyde (10 kDa, NOF, Japan), the triple agonists and the maleimide-PEG-aldehyde were reacted at a molar ratio of 1:1-3, a protein concentration of 1 to 5 mg/mL, and a low temperature, for 0.5 to 3 hours. The reaction was conducted under an environment in which 20-60% isopropanol was added to 50 mM Tris buffer (pH 7.5). Upon completion of the reaction, the reaction solutions were applied to SP Sepharose HP (GE healthcare, USA) to purify triple agonist having mono-PEGylated cysteine. The purified long-acting conjugates have a structure in which a triple agonist peptide, a polyethylene glycol (PEG) linker, and an Fc dimer are covalently linked at a molar ratio of 1:1:1, wherein the PEG linker is linked to only one of the two polypeptide chains of the Fc dimer.

In the immunoglobulin Fc, two monomers having the amino acid sequence of SEQ ID NO: 123 (consisting of 221 amino acids) form a homodimer through an inter-disulfide bond between cysteines, which are the 3rd amino acids of the respective monomers, wherein the monomers of the homodimer independently form an inter-disulfide bond between the cysteines at positions 35 and 95 and an inter-disulfide bond between the cysteines at positions 141 and 199, respectively.

Then, the purified mono-PEGylated triple agonists and an immunoglobulin Fc were reacted at a molar ratio of 1:1-5, a protein concentration of 10-50 mg/ml, and 4 to 8°C for 12 to 18 hours. The reaction was conducted in an environment in which 10-50 mM sodium cyanoborohydride as a reducing agent and 10-30% isopropanol were added to 100 mM potassium phosphate buffer (pH 6.0). Upon completion of the reaction, the reaction solutions were applied to the butyl sepharose FF purification column (GE healthcare, USA) and Source ISO purification column (GE healthcare, USA) to purify conjugates including the triple agonists and immunoglobulin Fc.

The purity of the conjugates that was analyzed by reverse-phase chromatography, size exclusion chromatography, and ion exchange chromatography after the preparation was 95% or more.

In particular, a conjugate in which a triple agonist, obtained by amidation of the C-terminus of the triple agonist of SEQ ID NO: 21, is bound to the immunoglobulin Fc via PEG was named "conjugate including SEQ ID NO: 21 and immunoglobulin Fc" or "long-acting conjugate of SEQ ID NO: 21", and these may be used interchangeably herein.

In particular, a conjugate in which a triple agonist, obtained by amidation of the C-terminus of the triple agonist of SEQ ID NO: 22, is bound to the immunoglobulin Fc via PEG was named "conjugate including SEQ ID NO: 22 and immunoglobulin Fc" or "long-acting conjugate of SEQ ID NO: 22", and these may be used interchangeably herein.

In particular, a conjugate in which a triple agonist, obtained by amidation of the C-terminus of the triple agonist of SEQ ID NO: 42, is bound to the immunoglobulin Fc via PEG was named "conjugate including SEQ ID NO: 42 and immunoglobulin Fc" or "long-acting conjugate of SEQ ID NO: 42", and these may be used interchangeably herein.

In particular, a conjugate in which a triple agonist, obtained by amidation of the C-terminus of the triple agonist of SEQ ID NO: 43, is bound to the immunoglobulin Fc via PEG was named "conjugate including SEQ ID NO: 43 and immunoglobulin Fc" or "long-acting conjugate of SEQ ID NO: 43", and these may be used interchangeably herein

In particular, a conjugate in which a triple agonist, obtained by amidation of the C-terminus of the triple agonist of SEQ ID NO: 50, is bound to the immunoglobulin Fc via PEG was named "conjugate including SEQ ID NO: 50 and immunoglobulin Fc" or "long-acting conjugate of SEQ ID NO: 50", and these may be used interchangeably herein.

In particular, a conjugate in which a triple agonist, obtained by amidation of the C-terminus of the triple agonist of SEQ ID NO: 77, is bound to the immunoglobulin Fc via PEG was named "conjugate including SEQ ID NO: 77 and immunoglobulin Fc" or "long-acting conjugate of SEQ ID NO: 77", and these may be used interchangeably herein

In particular, a conjugate in which a triple agonist, obtained by amidation of the C-terminus of the triple agonist of SEQ ID NO: 96, is bound to the immunoglobulin Fc via PEG was named "conjugate including SEQ ID NO: 96 and immunoglobulin Fc" or "long-acting conjugate of SEQ ID NO: 96", and these may be used interchangeably herein.

### Experimental Example 1: In vitro activities of triple agonists and

### long-acting conjugates thereof

To measure the activities of the triple agonists and the long-acting conjugates thereof prepared in Examples 1 and 2, a method of measuring cell activity *in vitro* was employed by using cell lines in which GLP-1, glucagon (GCG), and GIP receptors were transformed, respectively.

The cell lines were obtained by transforming Chinese hamster ovary (CHO) cells to express human GLP-1 receptor, human GCG receptor, and human GIP receptor, respectively, and these cell lines were suitable for the measurement of the activities of GLP-1, GCG, and GIP. Therefore, the activities for the receptors were measured using the transformed cell lines, respectively.

For the measurement of the GLP-1 activity of the triple agonists and the long-acting conjugates thereof prepared in Examples 1 and 2, human GLP-1 was subjected to 4-fold serial dilution from 50 nM to 0.000048 nM, and the triple agonists and the long-acting conjugates thereof prepared in Examples 1 and 2 were subjected to 4-fold serial dilution from 400 nM to 0.00038 nM. The cultures were removed from the cultured CHO cells expressing the human GLP-1 receptor, and 5 µL of each of the continuously diluted materials was added to the cells, and then 5 µL of a cAMP antibody-containing buffer was added, followed by incubation at room temperature for 15 minutes. Then, 10 µL of a detection mix containing a cell lysis buffer was added to lyse the cells, followed by incubation at room temperature for 90 minutes. The cell lysates upon completion of the reaction were applied to the LANCE cAMP kit (PerkinElmer, USA) to calculate EC₅₀ values through accumulated cAMP, and then the values were compared with each other. The relative potency ratios compared with human GLP-1 are shown in Tables 2 and 3 below.

For the measurement of the GCG activity of the triple agonists and the long-acting conjugates thereof prepared in Examples 1 and 2, human GCG was subjected to 4-fold serial dilution from 50 nM to 0.000048 nM, and the triple agonists and the long-acting conjugates thereof prepared in Examples 1 and 2 were subjected to 4-fold serial dilution from 400 nM to 0.00038 nM. The cultures were removed from the cultured CHO cells expressing the human GCG receptor, and 5 µL of each of the continuously diluted materials was added to the cells, and then 5 µL of a cAMP antibody-containing buffer was added, followed by incubation at room temperature for 15 minutes. Then, 10 µL of a detection mix containing a cell lysis buffer was added to lyse the cells, followed by incubation at room temperature for 90 minutes. The cell lysates upon completion of the reaction were applied to the LANCE cAMP kit (PerkinElmer, USA) to calculate EC₅₀ values through accumulated cAMP, and then the values were compared with each other. The relative potency ratios compared with human GCG are shown in Tables 2 and 3 below.

For the measurement of the GIP activity of the triple agonists and the long-acting conjugates thereof prepared in Examples 1 and 2, human GIP was subjected to 4-fold serial dilution from 50 nM to 0.000048 nM, and the triple agonists and the long-acting conjugates thereof prepared in Examples 1 and 2 were subjected to 4-fold serial dilution from 400 nM to 0.00038 nM. The cultures were removed from the cultured CHO cells expressing the human GIP receptor, and 5 µL of each of the continuously diluted materials was added to the cells, and then 5 µL of a cAMP antibody-containing buffer was added, followed by incubation at room temperature for 15 minutes. Then, 10 µL of a detection mix containing a cell lysis buffer was added to lyse the cells, followed by incubation at room temperature for 90 minutes. The cell lysates upon completion of the reaction were applied to the LANCE cAMP kit (PerkinElmer, USA) to calculate EC₅₀ values through accumulated cAMP, and then the values were compared with each other. The relative potency ratios compared with human GIP are shown in Tables 2 and 3 below.

**TABLE 2**

| Relative potency ratios of triple agonists | | | |
|---|---|---|---|
| SEQ ID NO | *In vitro* activity (%) compared with native peptide | | |
| | vs GLP-1 | vs Glucagon | vs GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | < 0.1 | < 0.1 | < 0.1 |
| 14 | 28.0 | < 0.1 | < 0.1 |
| 15 | 79.2 | <0.1 | <0.1 |
| 16 | 2.1 | < 0.1 | < 0.1 |
| 17 | 0.2 | < 0.1 | < 0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | < 0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | < 0.1 | < 0.1 |
| 46 | 1.4 | < 0.1 | < 0.1 |
| 47 | 2.4 | < 0.1 | < 0.1 |
| 48 | 1.5 | < 0.1 | < 0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**TABLE 3**

| Relative potency ratios of long-acting conjugates of triple agonists | | | |
|---|---|---|---|
| Long-acting conjugates | *In vitro* activity (%) compared with native peptide | | |
| | vs GLP-1 | vs Glucagon | vs GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

The long-acting conjugates of triple agonists prepared above have the function as a triple agonist, which can activate all of GLP-1, GIP, and glucagon receptors, and thus can be used as a medicine for multiple sclerosis.

### Experimental Example 2: Therapeutic effect of long-acting conjugate of triplet agonist for multiple sclerosis

The therapeutic effect of the triple agonist of the present invention was examined in a multiple sclerosis animal model that has been experimentally induced. The long-acting conjugate of the triple agonist of SEQ ID NO: 42 (long-acting conjugate of SEQ ID NO: 42) was selected as a representative example of a long-acting conjugate of a triple agonist and then experimented.

The multiple sclerosis animal model was constructed by administering proteolipid protein (PLP) and pertussis toxin (PTX) to SJL female mice. Symptom scores were categorized into 0 to 5 points based on the degree of multiple sclerosis progression in the animal model. Scoring was based on: 0 points for no multiple sclerosis symptoms; 1 point for limp tail; 2 points for impaired righting reflex; 3 points for partial paralysis of hind limbs; 4 points for hind limb paralysis; and 5 points for moribund. Symptoms of multiple sclerosis began to appear about 7 to 10 days after the administration of PLP/PTX, and the most severe symptoms, with an average score of 3 points, were shown on about day 14, and the symptoms lasted for about 10 days. Thereafter, the symptoms were mitigated for about 10 days and then relapsed again.

During the experiment, the mice were housed in groups and had free access to water. Test groups were assigned into Group 1 (normal animal - vehicle control group), Group 2 (multiple sclerosis - vehicle control group), Group 3 (multiple sclerosis - long acting conjugate of SEQ ID NO: 42 (1.4 nmol/kg), administered from Day 1), and Group 4 (multiple sclerosis - long acting conjugate of SEQ ID NO: 42 (1.4 nmol/kg), administered from Day 17). The vehicle and long-acting conjugate of SEQ ID NO: 42 were administered subcutaneously once every two days. The experiment was terminated on the 37th day. Statistical analysis was performed by two-way ANOVA.

### (1-1) Effect of administration for purpose of prevention

For the purpose of prevention of multiple sclerosis, the long-acting conjugate of SEQ ID NO: 42 was administered from Day 1 after model construction.

As a result, as can be seen in FIG. 1, the symptom score in the multiple sclerosis model was increased due to the administration of PLP/PTX, and the symptom scores in the first symptom appearing section and the subsequent relapse section were significantly reduced in the group administered the long-acting conjugate of SEQ ID NO: 42. The relapse of symptoms occurred on day 24 of administration in the multiple sclerosis model administered vehicle, while the relapse of symptoms began on day 28 in the group administered the long-acting conjugate of SEQ ID NO: 42, indicating that the long-acting conjugate delayed the time of relapse.

### (1-2) Effect of administration for purpose of treatment

For the purpose of treatment of multiple sclerosis, the long-acting conjugate of SEQ ID NO: 42 was administered from Day 17 after model construction.

As a result, as can be seen in FIG. 2, the symptom scores in the multiple sclerosis model due to the administration of PLP/PTX were similarly increased in the first symptom appearing section, while the symptom score was significantly reduced in the relapse section after Day 17 on which the administration of the long-acting conjugate of SEQ ID NO: 42 was began.

It can be therefore seen that the long-acting conjugate of SEQ ID NO: 42, which was a representative conjugate of a triple agonist of the present invention, had an effect of preventing and treating multiple sclerosis.

The above results support that the conjugates of triple agonists of the present invention can effectively treat multiple sclerosis and can be provided as novel medicines for multiple sclerosis. The above results especially indicate that the triple agonist conjugates can delay the onset of multiple sclerosis when used for the purpose of prevention and can significantly treat multiple sclerosis to be close to normal when used for the purpose of treatment.

While the present invention has been described with reference to the particular illustrative embodiments, a person skilled in the art to which the present invention pertains can understand that the present invention may be embodied in other specific forms without departing from the technical spirit or essential characteristics thereof. In this regard, the embodiments described above should be understood to be illustrative rather than restrictive in every respect. The scope of the invention should be construed that the meaning and scope of the appended claims rather than the detailed description and all changes or variations derived from the equivalent concepts fall within the scope of the present invention.

## Claims

1. A pharmaceutical composition for prevention or treatment of multiple sclerosis, the pharmaceutical composition comprising:
a pharmaceutically acceptable vehicle; and
a therapeutically effective amount of a peptide containing an amino acid sequence of any one of SEQ ID NOS: 1 to 102.

2. The pharmaceutical composition according to claim 1, wherein the peptide is in the form of a long-acting conjugate and the long-acting conjugate is represented by Formula 1 below:
[Formula 1] X-L-F
wherein X represents a peptide containing an amino sequence of any one of SEQ ID NOS: 1 to 102;
L represents a linker containing ethylene glycol repeat units;
F represents an immunoglobulin Fc region; and
"-" symbols represent covalent linkages between X and L and between L and F, respectively.

3. The pharmaceutical composition according to claim 1 or 2, wherein the C-terminus of the peptide is amidated.

4. The pharmaceutical composition according to claim 1 or 2, wherein the peptide contains an amino acid sequence selected from the group consisting of SEQ ID NOS: 21, 22, 42, 43, 50, 64, 66, 67, 70, 71, 76, 77, 96, 97, and 100.

5. The pharmaceutical composition according to claim 4, wherein the peptide contains an amino acid sequence selected from the group consisting of SEQ ID NOS: 21, 22, 42, 43, 50, 66, 67, 77, 96, 97, and 100.

6. The pharmaceutical composition according to claim 5, wherein the peptide contains an amino acid sequence selected from the group consisting of SEQ ID NOS: 21, 22, 42, 43, 50, 77, and 96.

7. The pharmaceutical composition according to claim 1 or 2, wherein the amino acids at positions 16 and 20 from the N-terminus in the sequence of the peptide form a ring.

8. The pharmaceutical composition according to claim 2, wherein L is polyethylene glycol.

9. The pharmaceutical composition according to claim 2, wherein the formula weight of an ethylene glycol repeat unit moiety in L is in a range of 1 to 100 kDa.

10. The pharmaceutical composition according to claim 2, wherein the immunoglobulin Fc region is aglycosylated.

11. The pharmaceutical composition according to claim 2, wherein F is an IgG Fc region.

12. The pharmaceutical composition according to claim 2, wherein the immunoglobulin Fc region is a dimer consisting of two polypeptide chains, and one end of L is linked only to one of the two polypeptide chains.

13. The pharmaceutical composition according to claim 2, wherein in the conjugate, one end of L is linked to F via a covalent linkage formed by reaction with an amine or thiol group of F, and the other end of L is linked to X via a covalent linkage formed by reaction with an amine or thiol group of X.

14. The pharmaceutical composition according to claim 1 or 2, wherein the multiple sclerosis is relapsing/remitting multiple sclerosis (RRMS), secondary progressive multiple sclerosis (SPMS), primary progressive multiple sclerosis (PPMS), or progressive relapsing multiple sclerosis (PRMS).
